# EUROPEAN PATENT SPECIFICATION

(11) **EP 3 283 889 B1**
(45) Date of publication and mention of the grant of the patent: **26.02.2020**
(21) Application number: 16721656.3
(22) Date of filing: 16.04.2016
(51) Int. Cl.: G01N 33/68

(54) **METHOD AND ASSAY FOR DIAGNOSING RAPIDLY PROGRESSIVE GLOMERULONEPHRITIS IN A SUBJECT**
VERFAHREN UND TEST ZUR DIAGNOSE VON RASCH PROGRESSIVER GLOMERULONEPHRITIS BEI EINER PERSON
PROCÉDÉ ET DOSAGE POUR LE DIAGNOSTIC RAPIDE DE LA GLOMÉRULONÉPHRITE ÉVOLUTIVE CHEZ UN SUJET

(30) Priority: 17.04.2015 GB 201506564; 22.04.2015 US 201562151302 P; 12.02.2016 EP 16155463
(43) Date of publication of application: 21.02.2018
(73) Proprietor: Euroimmun Medizinische Labordiagnostika AG, 23560 Lübeck (DE); The Provost, Fellows, Foundation Scholars, and the other members of Board, of the College of the Holy & Undiv. Trinity of Queen Elizabeth near Dublin, Dublin 2 (IE)
(72) Inventor: LITTLE, Mark, Dublin 2 (IE)
(86) International application number: PCT/EP2016/058471
(87) International publication number: WO 2016/166357

(56) References cited:
- TSUBOI NAOTAKE ET AL: "Association of Glomerular Macrophage Phenotypes and Urine Soluble CD163 with Disease Activity in Human Lupus Nephritis", ARTHRITIS & RHEUMATOLOGY (HOBOKEN), JOHN WILEY & SONS, INC, US, vol. 66, no. Suppl.10, 1 October 2014 (2014-10-01), page S727, XP009191726, ISSN: 2326-5191
- O'REILLY VINCENT P ET AL: "Urinary Soluble CD163 in Active Renal Vasculitis", JOURNAL OF THE AMERICAN SOCIETY OF NEPHROLOGY, LIPPINCOTT WILLIAMS & WILKINS, US, vol. 27, no. 9, 1 September 2016 (2016-09-01), pages 2906-2916, XP009191718, ISSN: 1533-3450, DOI: 10.1681/ASN.2015050511
- LONGXIANG SU ET AL: "Diagnostic value of urine sCD163 levels for sepsis and relevant acute kidney injury: a prospective study", BMC NEPHROLOGY, BIOMED CENTRAL, LONDON, GB, vol. 13, no. 1, 26 September 2012 (2012-09-26), page 123, XP021120340, ISSN: 1471-2369, DOI: 10.1186/1471-2369-13-123
- IKEZUMI YOHEI ET AL: "Alternatively activated macrophages in the pathogenesis of chronic kidney allograft injury", PEDIATRIC NEPHROLOGY, SPRINGER VERLAG, BERLIN, DE, vol. 30, no. 6, 9 December 2014 (2014-12-09), pages 1007-1017, XP035490414, ISSN: 0931-041X, DOI: 10.1007/S00467-014-3023-0 [retrieved on 2014-12-09]
- YING ZHANG ET AL: "The influence of cathelicidin LL37 in human anti-neutrophils cytoplasmic antibody (ANCA)-associated vasculitis", ARTHRITIS RESEARCH AND THERAPY, BIOMED CENTRAL, LONDON, GB, vol. 15, no. 5, 24 October 2013 (2013-10-24), page R161, XP021169097, ISSN: 1478-6354, DOI: 10.1186/AR4344

## Description

### Field of the Invention

The invention relates to determining the presence or absence of kidney failure in a subject. More specifically, the invention relates to a method and assay for determining the presence or absence of recurrence or flare of Rapidly Progressive Glomerulonephritis (RPGN)/crescentic Glomerulonephritis (CGN) in an individual.

### Background to the Invention

Rapidly progressive glomerulonephritis (RPGN)/crescentic Glomerulonephritis (CGN) causes kidney failure due to glomerular "crescent" formation. These "crescents", a hallmark of severe inflammatory injury, are infiltrates of inflammatory leukocytes (primarily macrophages) and proliferating intrinsic renal cells. The main causes of RPGN/CGN are small vessel vasculitis (usually with anti-neutrophil cytoplasm antibodies, ANCA, or anti-glomerular basement membrane, GBM, antibodies) and immune complex glomerulonephritis (due to systemic lupus erythematosus, SLE/lupus, or IgA nephropathy). Untreated, they result in irreversible complete loss of kidney function. Glomerulonephritis is the second most frequent cause of end-stage kidney disease, which requires permanent dialysis therapy or transplantation.

In the developed world, one in five adults and one in three children experience an episode of acute kidney injury (AKI) during a hospital admission, of whom about 5% will have CGN. Although most episodes of AKI respond to conservative measures, those with CGN need urgent specific treatment. Levels of soluble CD163 (sCD163) in urine have been associated with identifying AKI in patients with sepsis, but the values were not significant and this study was limited to the intensive care unit setting (Longxiang Su et al. BMC Nephrology, 2012, 13:123).

Diagnosis of these conditions is aided by well-established serological biomarkers and kidney biopsy when required. However, there is an unmet need that lies within specific elements of the clinical care of these patients. Most forms of RPGN follow a relapsing and remitting course. In particular, in established ANCA-vasculitis and lupus nephritis, the treating physician is often faced with a clinical decision when faced with non-specific symptoms and change in kidney function. In this setting, the serological markers used in diagnosis have limited utility, so the physician must rely on detection of urinary blood, protein and casts, in association with non-specific inflammation markers such as c-reactive protein. Kidney biopsy, an invasive, expensive and potentially dangerous investigation, is often required.

Personalised guidance of immunosuppressive therapy intensity using a biomarker providing direct evidence of glomerular macrophage activation also faces similar problems. Tracking the degree of glomerular inflammation and destruction during a course of potentially toxic induction treatment is currently undertaken using a "one-size fits all" approach, or using best guesses based on urine parameters and excretory kidney function.

Tsuboi et al. (Tsuboi, N. et al., 2014, Arthritis & Rheumatology, vol. 66, no. Suppl. 10, page S727) disclose that urine sCD163 level has a potential significance for estimation of disease activity in human lupus nephritis (LN) patients.

Ying et al. (Ying, Z. et al., 2013, Arthritis research and therapy, vol. 15, no. 5, page R161) disclose that increased expression of LL37 and interferon-α are associated with human anti-neutrophils cytoplasmic antibody (ANCA)-associated vasculitis.

It is the object of the invention to overcome at least one of the above-referenced problems.

### Summary of the Invention

It is an object of the invention to provide a simple non-invasive test that would distinguish active glomerulonephritis from mimics, such as ischemic renal injury, interstitial nephritis, other de-novo renal injury {e.g. secondary to paraprotein deposition) or urine infection, which would be valuable in clinical practice.

A non-invasive point of care test would allow optimisation of treatment duration, personalising treatment with the goal of minimising unnecessary exposure to toxic induction treatment or ensuring complete resolution of inflammation before switching to maintenance therapy. Treatment of CGN, particularly when caused by ANCA vasculitis or anti-GBM disease, usually comprises intense immunosuppression with corticosteroids and approximately 3 months of cyclophosphamide or treatment with rituximab (a chimeric monoclonal antibody against the protein CD20, sold under the trade names Rituxan®, MabThera® and Zytux®). Current biomarkers that guide the intensity of this therapy include urine dipstick (for protein and blood) and serum creatinine level. It has been reported that dipstick haematuria also takes a similarly long time to resolve and that its persistence does not predict final outcome, with 40% still displaying haematuria at the time of switching to maintenance immunosuppression (Chen, T.K., et al., Semin Arthritis Rheum, 2014. 44(2): p. 198-201). These findings suggest that at the point of de-escalation of immunosuppression, kidney function is generally still improving by conventional measures. Therefore, these measures cannot be considered as useful guides to the timing of, for example, discontinuation of cyclophosphamide therapy.

In accordance with the invention, there is provided, as set out in the appended claims, a method for identifying an individual with active crescentic glomerulonephritis (CGN), the method comprising the step of assaying a biological sample obtained from the individual for the presence of an elevated level of sCD163 when compared to levels in a reference sample, wherein an elevated level of sCD163 when compared to levels in a reference sample correlates with the individual having active CGN, wherein the biological sample is urine, and the CGN is caused by small vessel vasculitis (SVV) or anti-glomerular basement membrane (anti-GBM) antibody disease.

In one embodiment, the method further comprises the step of assaying the biological sample obtained from the individual for the presence of an elevated level of monocyte chemoattractant protein- 1 (MCP-1) when compared to levels in a reference sample, wherein an elevated level of MCP-1 when compared to levels in a reference sample, in combination with an elevated level of sCD163 when compared to levels in a reference sample, correlates with the individual having active CGN.

In accordance with the invention, there is provided, as set out in the appended claims, a method for assessing whether an individual should continue to receive treatment for CGN, the method comprising the step of assaying a biological sample obtained from the individual for the presence of an elevated level of sCD163 when compared to levels in a reference sample, wherein an elevated level of sCD163 when compared to levels in a reference sample correlates with the individual being suitable for continuance of the treatment, wherein the biological sample is urine, and the CGN is caused by small vessel vasculitis (SVV) or anti-glomerular basement membrane (anti-GBM) antibody disease.

Preferably, the method further comprises the step of assaying the biological sample obtained from the individual for the presence of an elevated level of monocyte chemoattractant protein- 1 (MCP-1) when compared to levels in a reference sample, wherein an elevated level of MCP-1 when compared to levels in a reference sample, in combination with an elevated level of sCD163 when compared to levels in a reference sample, correlates with the individual being suitable for continuance of the treatment.

Preferably, the treatment consists of glucocorticoid treatment and/or immunosuppressant treatment.

In accordance with the invention, there is provided, as set out in the appended claims, a method for distinguishing CGN flare from other causes of clinical decline in an individual known previously to have CGN or a systemic autoimmune disease typically associated with CGN, the method comprising the step of assaying a biological sample obtained from the individual for the presence of an elevated level of sCD163 when compared to levels in a reference sample, wherein an elevated level of sCD163 when compared to levels in a reference sample correlates with the individual suffering from CGN flare and not from other causes of clinical decline, wherein the biological sample is urine, and the CGN is caused by small vessel vasculitis (SVV) or anti-glomerular basement membrane (anti-GBM) antibody disease.

Preferably, the method further comprises the step of assaying the biological sample obtained from the individual for the presence of an elevated level of monocyte chemoattractant protein- 1 (MCP-1) when compared to levels in a reference sample, wherein an elevated level of MCP-1 when compared to levels in a reference sample, in combination with elevated levels of sCD163 when compared to levels in a reference sample, correlates with the individual suffering from CGN flare and not from other causes of clinical decline.

Preferably, the other causes of clinical decline comprise ischemic renal injury, interstitial nephritis, secondary to paraprotein deposition, drug toxicity, myoglobinuric tubular injury, malignancy, acute tubular necrosis, urine infection, haemolytic uremic syndrome/thrombotic thrombocytopenic purpura or fibrillary glomerulonephritis.

Further described herein is that the biological sample is selected from the group consisting of urine, serum, blood, and kidney biopsy.

Preferably, the reference sample is an individual identified as having kidney disease not caused by SVV, acute active CGN or other forms of CGN.

Further described herein is that the CGN flare is caused by small vessel vasculitis (with and without the presence of anti-neutrophil cytoplasm antibodies (ANCA)), anti- glomerular basement membrane (anti-GBM) antibody disease and immune complex glomerulonephritis, including systemic lupus erythematosus - SLE/lupus, cryoglobulinemia, post-infectious glomerulonephritis, fibrillary glomerulonephritis, mesangiocapillary glomerulonephritis, C3 glomerulopathy or IgA nephropathy.

In one embodiment, there is provided, as set out in the appended claims, glucocorticoid or immunosuppressive therapy for use in a method for treating an individual with established crescentic glomerulonephritis (CGN) or recurrence of CGN, the method comprising the steps of assaying a biological sample obtained from the individual for the presence of an elevated level of sCD163 when compared to levels in a reference sample, identifying the individual for treatment if the biological sample shows an elevated level of sCD163 when compared to levels in a reference sample, and treating the individual with a glucocorticoid and/or an immunosuppressive therapy, wherein the biological sample is urine, the CGN is caused by small vessel vasculitis (SVV) or anti-glomerular basement membrane (anti-GBM) antibody disease, the glucocorticoid is selected from prednisolone, methyl-prednisolone, hydrocortisone or dexamethasone, and immunosuppressive therapy is selected from cyclophosphamide, rituximab, mycophenolate mofetil, azathioprine or methotrexate.

Preferably, the method further comprises the step of assaying a biological sample obtained from the individual for the presence of an elevated level of monocyte chemoattractant protein- 1 (MCP-1) when compared to levels in a reference sample, identifying the individual for treatment if the biological sample shows an elevated level of MCP-1 when compared to levels in a reference sample, in combination with an elevated level of sCD163 when compared to levels in a reference sample, and treating the individual with a glucocorticoid and/or an immunosuppressive therapy.

Further described herein is a system for carrying out the method as described above, the system comprising: a determination module; and a display module; wherein the determination module is configured to receive at least one test sample of an individual and perform at least one test analysis on the test sample to generate sCD163 data indicating the levels of sCD163 levels when compared to levels in a reference sample; and wherein the display module displays the sCD163 levels.

The determination module is configured to receive at least one test sample of an individual and perform at least one test analysis on the test sample to generate MCP-1 data and sCD163 data indicating the levels of MCP-1 and sCD163 levels when compared to levels in a reference sample; and wherein the display module displays the MCP-1 and sCD163 levels

The system further comprises a storage device, wherein the storage device stores the sCD163 data generated by the determination module. The system further comprises a storage device, wherein the storage device also stores the MCP- 1 data generated by the determination module.

The system further comprises a correlation module, wherein the correlation module identifies the individual with CGN or experiencing CGN flare if the sCD163 data from the test sample shows an increased level of sCD163 when compared to levels in a reference sample. The correlation module identifies the individual with CGN or experiencing CGN flare if the sCD163 and MCP-1 data from the test sample shows an increased level of sCD163 and MCP-1 when compared to levels in a reference sample.

In one embodiment, there is provided, as set out in the appended claims, a method comprising treating an individual with glucocorticoid and/or immunosuppressive therapy, wherein the individual has previously been identified as having CGN or experiencing CGN flare based on the presence of an elevated level of sCD163 in a biological sample from the individual when compared to levels in a reference sample.

Preferably, the individual has previously been identified as having CGN or experiencing CGN flare based on the presence of an elevated level of MCP-1 in the biological sample from the individual when compared to levels in a reference sample, in combination with an elevated level of sCD163 in the biological sample from the individual when compared to levels in a reference sample.

In one embodiment of the methods described above, the assaying step employs an in vitro diagnostic apparatus configured to detect an elevated level of a marker selected from sCD163, and optionally MCP-1, in urine, and in which the method comprises a step of assaying the biological sample with the in vitro diagnostic apparatus.

The in vitro diagnostic apparatus comprises an antibody or antibody fragment capable of specifically binding to a protein marker selected from sCD163 and optionally MCP-1 immobilised to a support, in which the method comprises the steps of contacting the biological sample with the antibody or antibody fragment under conditions that allow an immuno specific protein-antibody or protein-antibody fragment binding reaction to occur, and detection of an immuno specific protein-antibody or protein-antibody fragment binding reaction.

The in vitro diagnostic apparatus is selected from an ELISA kit and a dipstick kit.

The in vitro diagnostic apparatus is a dipstick kit comprising: a dipstick having the antibody or antibody fragment capable of specifically binding to sCD163 and optionally MCP-1 immobilised thereon; one or more reagents capable of detecting the immuno specific protein-antibody or protein-antibody fragment binding reaction and emitting a detectable signal; and a correlating device for correlating the detectable signal with an elevated level of sCD163 and optionally MCP-1.

Ideally, the detectable signal is a colour change and in which the correlating device is a colour chart.

The Applicant suggests that the urine sCD 163 level reflects the activity of CGN and thus provides a specific biomarker for both the diagnosis of CGN flare, and for tracking the glomerular response to induction treatment (thus adjusting treatment accordingly).

The pattern of urine sCD163 excretion over time in patients treated for RPGN could be determined using the method of the invention and also used to assess urine sCD163 excretion values in RPGN mimics, such as urinary tract infection and tubulointerstitial nephritis.

### Brief Description of the Drawings

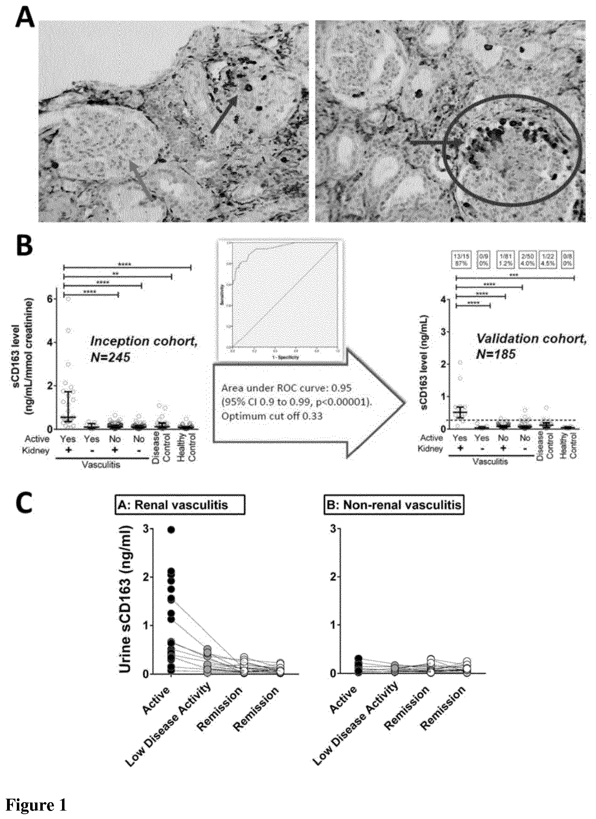

The invention will be more clearly understood from the following description of an embodiment thereof, given by way of example only, with reference to the accompanying drawings, in which:-
**Figure 1** illustrates data supporting the use of urine sCD163 as a biomarker of active renal vasculitis. A: Immunohistochemical stain of kidney tissue from a patient presenting with renal vasculitis. CD163 bearing macrophages stain brown and are predominantly located within an injured glomerulus (red arrow, left panel), which lies adjacent to an uninjured glomerulus (green arrow). The urinary space within Bowman's capsule (red arrow, right panel) lies adjacent to an area of crescent formation (red oval) thereby allowing shedding of CD163 directly into the urine. B: Testing of urine sCD163 as a biomarker of active renal vasculitis in two cohorts of patients and controls. Note that values are normalised to creatinine; actual measured values were between 1 and 30ng/mL. The inception cohort was used to define an optimal cut-off level of 0.33ng/mL/mmol creatinine. C: Serial testing of urine sCD163 as patients are treated and enter remission. The levels fall rapidly and remain low across two remission time points.

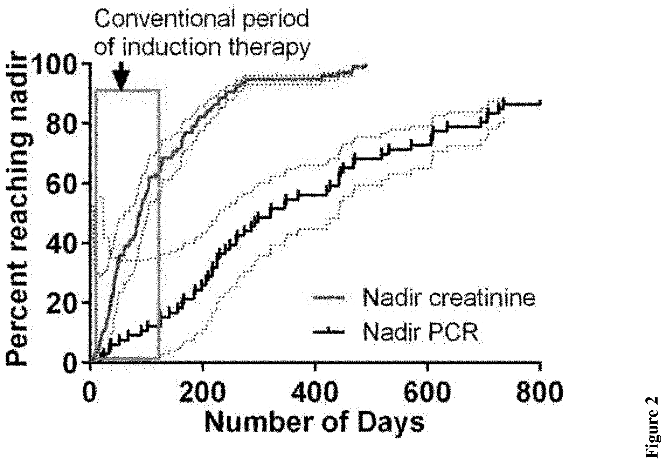

**Figure 2** illustrates Kaplan Meier curves depicting time to reaching nadir creatinine and urine protein:creatinine ratio (PCR). Dotted lines indicate 95% confidence interval.

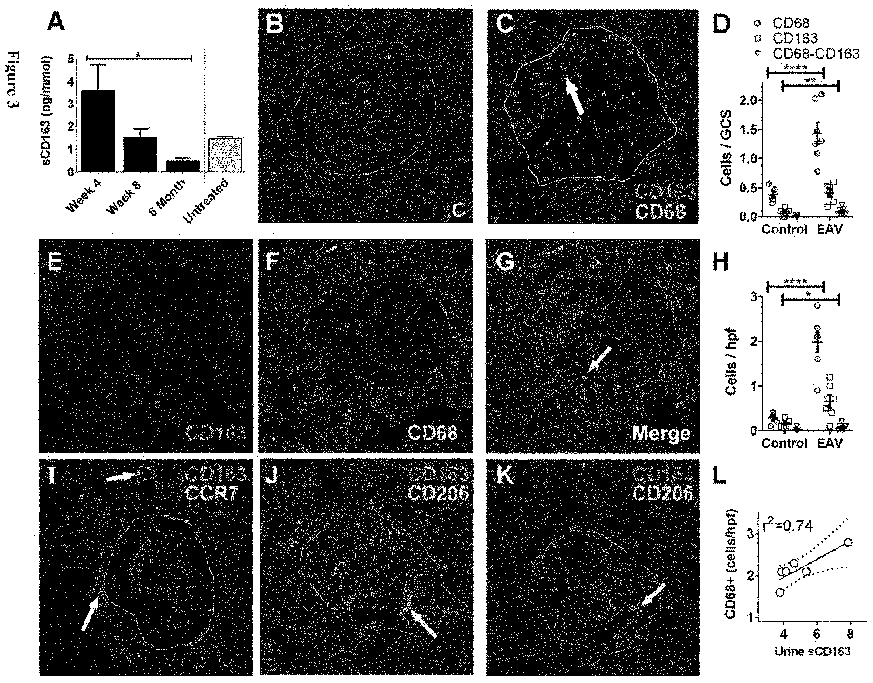

**Figure 3** illustrates that CD163 is present in the urine and kidneys of rats with experimental autoimmune vasculitis (EAV). **A.** Urine was collected from healthy control rats (n=3) and rats 28 days (n=5), 56 days (n=6) and 186 days (n=3) after induction of EAV. sCD163 levels were determined by ELISA. Data are presented as mean sCD163 ng/mmol ± standard error of the mean. One-way ANOVA and Dunn's multiple comparison test were used to test for linear trend (*p<0.05). **B,C,E,F,G,I,J,K.** Confocal images of glomeruli from rats with EAV dual stained with antibodies to the macrophage marker CD68 (C,F,G, green) and CD163 (red). Panels B and C depict representative images from tissue stained with isotype control and test antibody respectively. The white line indicates Bowman's capsule, while the fine yellow line in panel C indicates a glomerular crescent (white arrow). Panels E and F show confocal images in the CD163 and CD68 channels respectively, while panel G shows the merged image. Although most cells are positive for one of the markers only, cells are also identified with dual staining (yellow arrow). Panel I shows dual staining with CD163 (yellow arrow) and CCR7 (green); there was no co-localisation between the 2 markers, CCR7 being most strongly expressed in small blood vessels (white arrow). Panels J and K show staining with CD163 and CD206 (green), showing co-localisation in panel J (arrow) and lack of co-localisation in panel K (arrow). **D,H.** Using 5uM kidney sections from EAV and control rats, cells staining positive for CD68, CD163 or both markers were counted blind in 30 consecutive glomeruli **(D)** and 10 40x fields containing tubulointerstitial compartment only **(H),** (*p<0.05, **p<0.01, ****p<0.001). **L.** The average number of cells per high power field (hpf) in rats with EAV was correlated with the urine sCD163 level in the same animal.

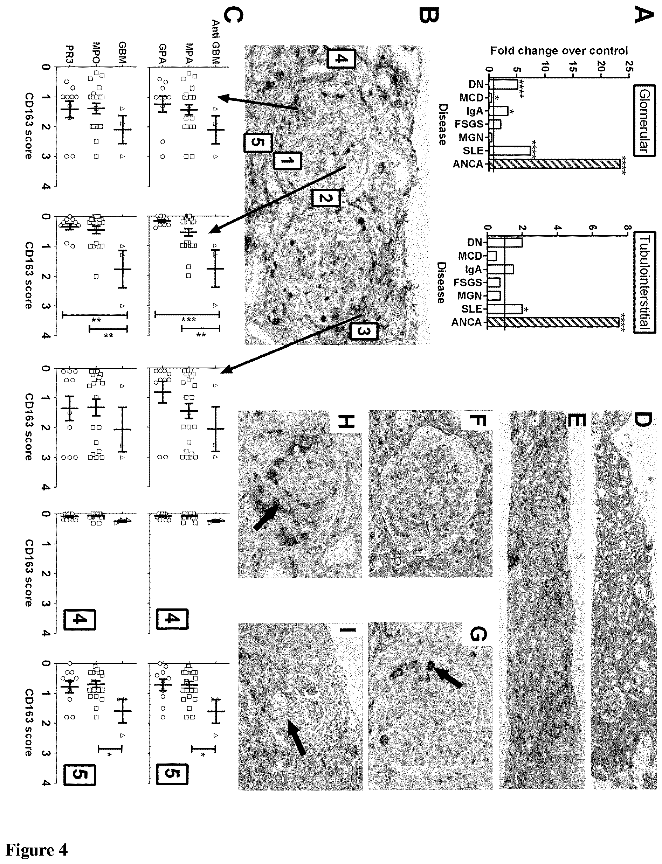

**Figure 4** illustrates that CD163 is highly expressed in the kidneys of patients with vasculitis. A. RNA was extracted from microdissected glomerular and tubulointerstitial compartments from patients with diabetic nephropathy (DN), minimal change disease (MCD), IgA nephropathy (IgA), focal and segmental glomerulosclerosis (FSGS), membranous glomerulonephritis (MGN), lupus nephritis (SLE) and ANCA vasculitis. The degree of expression of the CD163 gene compared to microdissected healthy control kidney was determined by Affymetrix microarrays. Bars represent fold changes compared to the respective controls (LDs). (****q<0.01%; *q<5%) **B.** Paraffin-embedded human kidney sections from patients with vasculitis were stained for CD163 protein by immunohistochemistry and scored blind according to the location of cells with each of 5 regions: (1) within regions of fibrinoid necrosis or crescent formation, (2) within regions of apparently normal glomeruli, (3) in the peri-glomerular region, (4) within tubules and (5) in the interstitial compartment. C. CD163 scores in each of the respective 5 regions stratified by clinical diagnosis (upper graphs) and antibody specificity (lower graphs), (*p<0.05, **p<0.01, ***p<0.001). **D-I.** Images depict representative low power (40x magnification) views of healthy control (D) and vasculitic (E) kidney, alongside high power (400X) views of healthy control kidney (F), a glomerulus with mild vasculitic injury (G, arrow), a severely affected glomerulus with established crescent formation (H, arrow) and a glomerulus with a fibrous crescent from previous vasculitic injury (I, arrow, 200X)..

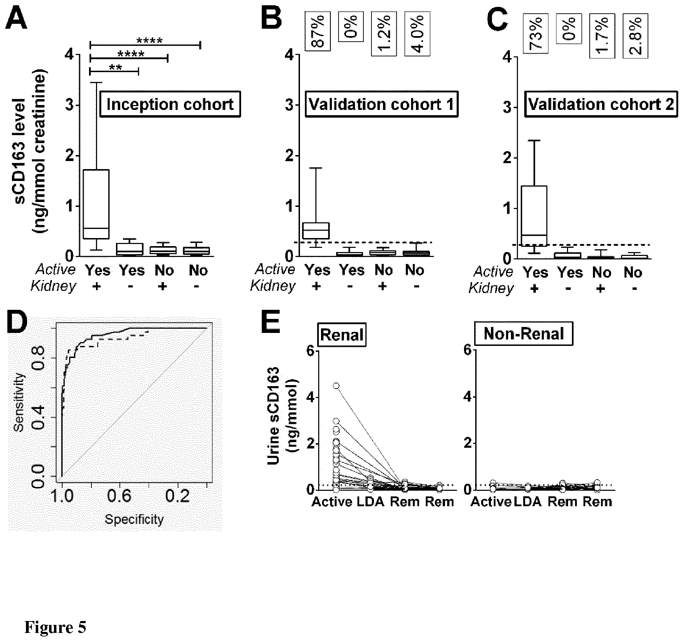

**Figure 5** illustrates that sCD163 is elevated in the urine of those with active renal vasculitis but not active extra-renal vasculitis. sCD163 levels were measured by ELISA in an inception cohort (n=177) **(A)** and two validation cohorts (n=155 + 133) **(B,C).** Graphs show comparison of levels found in active renal vasculitis, active extra-renal vasculitis, remission (Rem) renal vasculitis and remission extra-renal vasculitis. Data are presented as median sCD163 ng/mmol creatinine with interquartile range. The boxes in B and C indicate the fraction of positive samples in each group using the optimum cut-off of 0.3ng/mmol. Non-parametric one-way ANOVA (Kruskal-wallis test) and Dunn's multiple comparison test were used to test for significance of each group compared to the active renal vasculitis group (**p<0.01, ***p<0.001, ****p<0.0001). **D.** ROC curves calculated from normalised (solid line) and non-normalised (dashed line) data derived from the inception cohort depicting the ability of sCD163 to detect active renal vasculitis. The respective areas under the curves were 0.94 and 0.96 respectively. **E.** sCD163 was measured by ELISA in serial samples during periods of active disease, low disease activity (LDA) and remission (Rem). Graphs show sCD163 levels over time in renal and extra-renal vasculitis patients. Lines depict samples from the same subject at different time points.

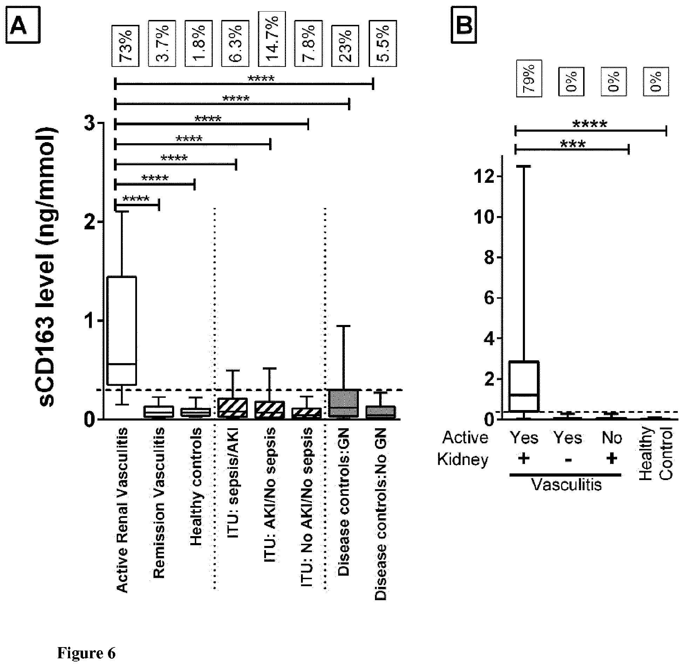

**Figure 6** illustrates that sCD163 is elevated in the urine of patients with active renal vasculitis and compared to a range of control groups. **(A)** The data from the inception and validation cohorts (n=465) were combined and compared to healthy controls (n=55) and a diverse disease control group comprising samples from patients in the intensive care unit with sepsis with or without acute kidney injury (AKI) (n=32), AKI without sepsis (n=286) and no AKI or sepsis (n=463), in addition to a non-ICU disease control group with (n=30) and without (n=54) non-vasculitic glomerulonephritis (GN). The boxes refer to the fraction of cases with a urine sCD163 level >0.3ng/mmol (dotted line). **(B)** sCD163 levels were also measured by ELISA in an independent external validation cohort (n=52). Graph showing comparison of levels found in active renal vasculitis (Act), active extra-renal vasculitis, remission renal vasculitis (Rem) and healthy controls. Data are presented as median sCD163 ng/mmol creatinine with interquartile range. Non-parametric one-way ANOVA (Kruskal-wallis test) and Dunn's multiple comparison test were used to test for significance of each group compared to the active renal vasculitis group (***p<0.001, ****p<0.0001).

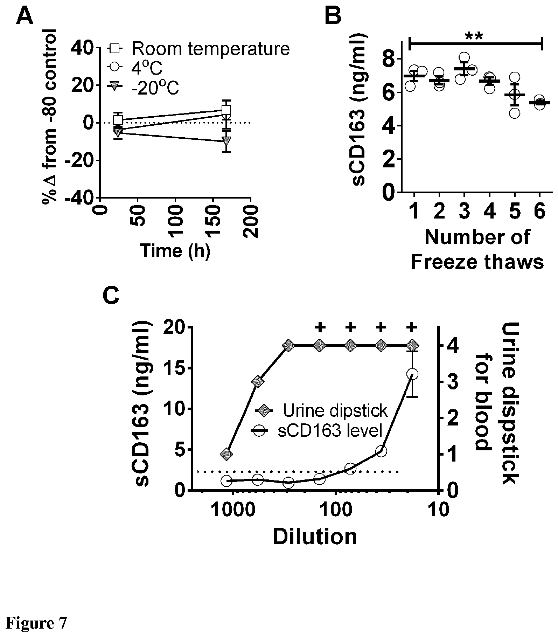

**Figure 7** illustrates that sCD163 is stable in urine across a range of temperatures and urine registering positive for blood using urine dipstick is negative for urine sCD163. A. Six samples of known sCD163 level were stored for 24 and 168 hours (one week) at room temperature, 4oC and -20oC. The % change in sCD163 level from samples stored in parallel at -80oC was plotted against time; mean±SEM. B. Samples were subjected to between 1 and 6 freeze-thaw cycles and sCD163 level determined by ELISA; mean±SEM. C. Healthy control urine was spiked with serial dilutions of blood and the level of sCD163 was determined by ELISA. The + symbols indicate those dilutions at which the urine appeared macroscopically blood stained. The dotted line indicates the upper limit of normal as determined in the inception cohort.

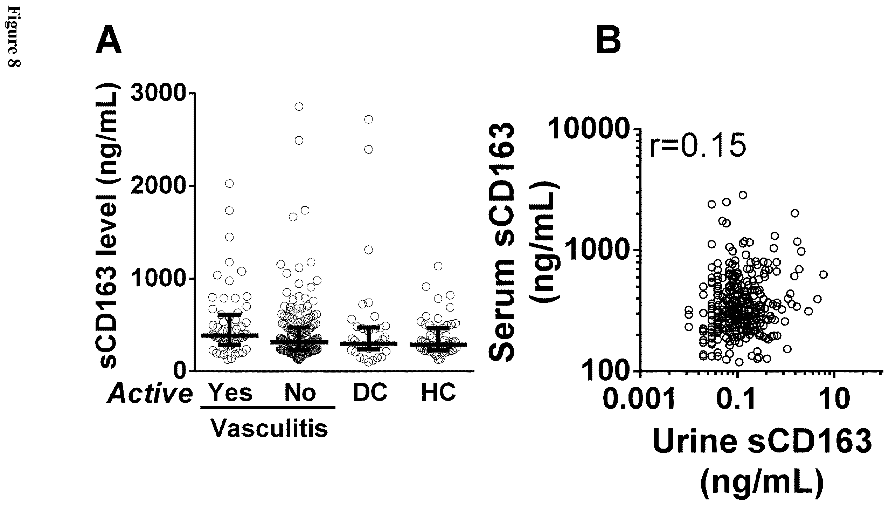

**Figure 8** illustrates that sCD163 levels are not elevated in the blood of patients with systemic vasculitis. A. sCD163 levels were determined in serum by ELISA and levels were compared between patients with active and remission disease as well as disease controls and healthy controls. Data are presented as median sCD163 ng/ml with interquartile range. Non-parametric one-way ANOVA (Kruskal-Wallis test) and Dunn's multiple comparison tests were used to test for significance of each group compared to the active vasculitis group. B. sCD163 levels were also determined in urine by ELISA and the degree of correlation between serum and urine levels were determined by Spearman's test (n=337).

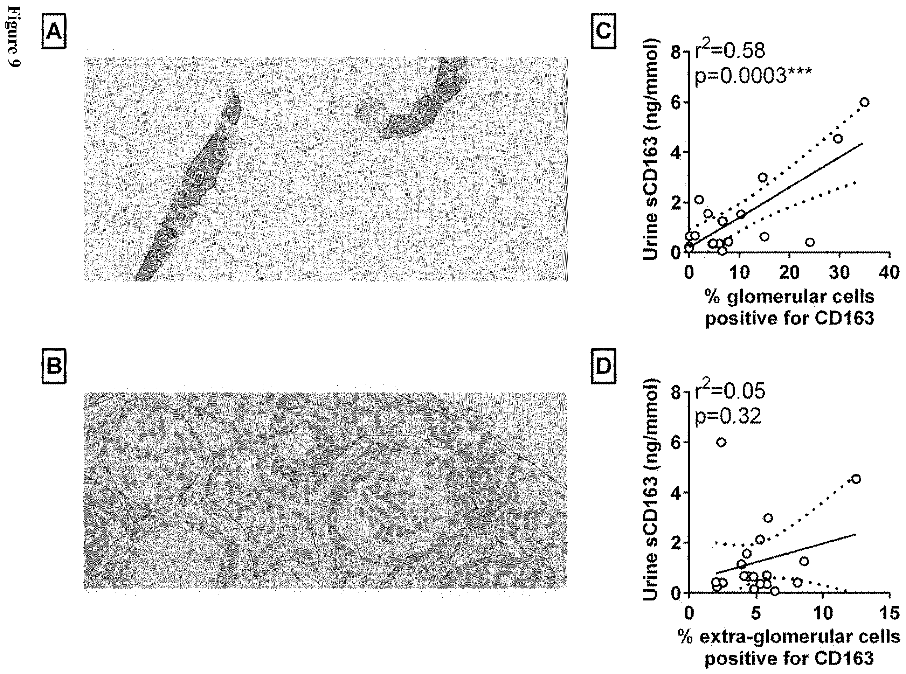

**Figure 9** illustrates that Urinary sCD163 levels correlate with glomerular CD163 staining but not extra-glomerular staining. (A) Renal tissue was stained for CD163 and separate areas were defined for glomeruli (red), obsolescent glomeruli (green) and extra-glomerular tissue (blue). (B) Vision analysis software was trained to differentiate between nuclei surrounded by DAB staining (yellow) and cells with no DAB staining (green). The level of normalised urine sCD163 was then correlated with the fraction of cells stained for CD163 in the glomerular (C) and extra-glomerular (D) compartments. ***p<0.005

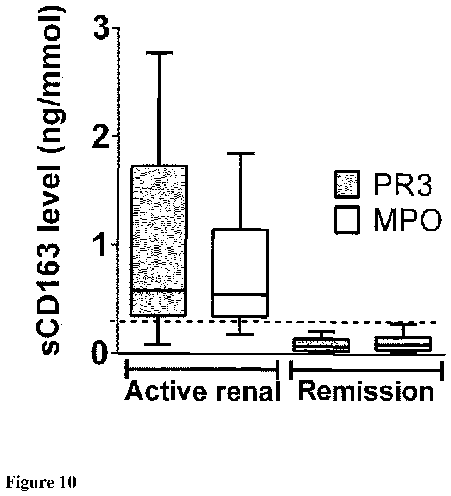

**Figure 10** illustrates that Urinary sCD163 levels are not different between MPO-ANCA (n=36 active, 113 remission) and PR3-ANCA (n=25 active, 190 remission) positive patients. Urinary sCD163 levels, as determined by ELISA and normalised to urine creatinine, were stratified by ANCA specificity.

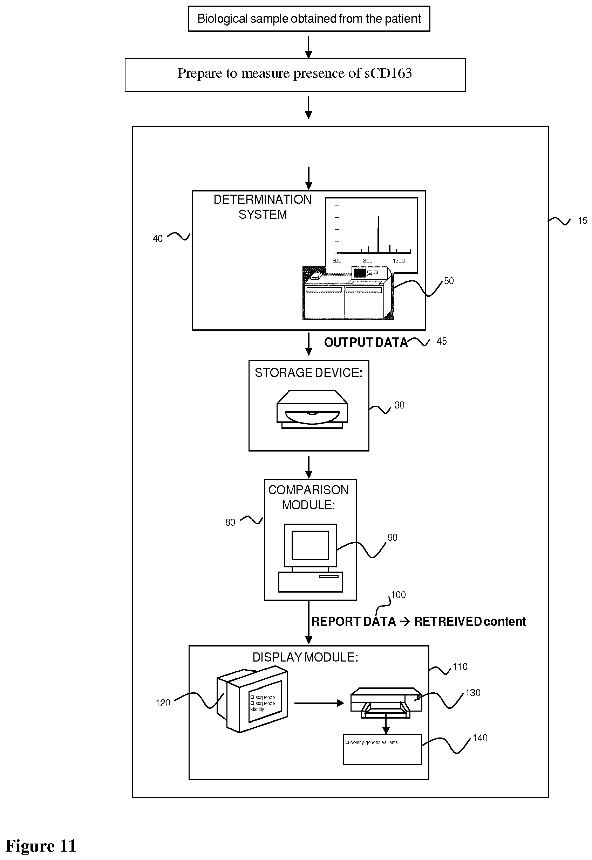

**Figure 11** is a flow diagram illustrating the functional modules of the system of the claimed invention.

### Detailed Description of the Drawings

The invention relates to a methods for identifying individuals that are likely to have crescentic glomerulonephritis (CGN) in undifferentiated acute kidney injury (AKI), or have recurrence of CGN, or a methods for identifying individuals in need of treatment for CGN or CGN flare. The identification of such individuals is useful as it allows a clinician to determine whether an individual presenting with AKI has CGN rather than multiple myeloma, haemolytic uraemic syndrome, acute tubular necrosis or other cause of decline in kidney function. These methods will accelerate this diagnostic process and help to avoid the requirement for kidney biopsy. Further, it also identifies whether individuals with, for example, ANCA vasculitis or lupus nephritis are suffering a renal relapse (CGN flare), which otherwise would require a renal biopsy. The invention also relates to a method for tracking glomerular inflammation in an individual being treated for CGN, with a view to guiding when treatment can be stopped. The methods estimate disease activity during the course of immunosuppressant (induction) treatment for CGN, thereby allowing a rational clinical decision about when to de-escalate therapy.

### Definitions

In the specification, the term "acute kidney injury" should be understood to mean a decline in glomerular filtration rate over a period of hours or days. It is the abrupt loss of kidney function, resulting in the retention of urea and other nitrogenous waste products and in the dysregulation of extracellular volume and electrolytes.

In the specification, the term "undifferentiated (community acquired) acute kidney injury" should be understood to mean acute kidney injury acquired outside hospital with unknown cause.

In the specification, the term "crescentic glomerulonephritis (CGN)", "rapidly progressive glomerulonephritis (RPGN)" and "CGN flare" should be understood to mean severe glomerular inflammation resulting in destruction of the glomerular tuft and accumulation of inflammatory cells around the tuft in the shape of a crescent. Flare of CGN indicates recurrence of CGN after a period of time in an individual known to have either prior CGN or a systemic autoimmune disease (such as ANCA vasculitis or SLE) in which one may expect CGN as part of the disease. The terms "crescentic glomerulonephritis (CGN)" and "rapidly progressive glomerulonephritis (RPGN)" may be used interchangeably.

In the specification, the term "small vessel vasculitis (SVV)" should be understood to mean an autoimmune disease characterised by destruction of small blood vessels, with resultant injury to the organ or tissue supplied by that vessel.

In the specification, the term "acute vasculitis" should be understood to mean vasculitis that is currently active, i.e. there is acute inflammation present in the region of the blood vessel wall, associated with evidence of organ dysfunction.

In the specification, the term "ANCA vasculitis" should be understood to mean small vessel vasculitis (SVV) in the setting of anti-neutrophil cytoplasm antibodies (ANCA).

In the specification, the term "CGN mimics" should be understood to mean other conditions that have clinical features similar to CGN such as ischemic renal injury, interstitial nephritis, secondary to paraprotein deposition, drug toxicity, myoglobinuric tubular injury, malignancy, acute tubular necrosis, urine infection, haemolytic uremic syndrome/thrombotic thrombocytopenic purpura or fibrillary glomerulonephritis.

In the specification, the term "anti-GBM disease" should be understood to mean small vessel vasculitis (SVV) in the setting of anti-glomerular basement membrane (GBM) antibodies.

In the specification, the term "disease controls" should be understood to mean individuals with kidney disease not due to small vessel vasculitis (SVV), CGN mimics, acute active CGN or other forms of CGN.

In the specification, the term "healthy control group" should be understood to mean individuals with no known kidney or other major disease. The normal level of sCD163 in a healthy control group is generally considered as 2 SD above and below the mean.

In the specification, the term "soluble CD163 (sCD163)" should be understood to mean the soluble (shed) form of CD163, a trans-membrane molecule on the surface of activated macrophages, the cell-type most frequently found in glomerular crescents. CD163 is traditionally considered a marker of M2c macrophages. This protein has been recognised in many studies as a macrophage activation marker, and it has recently been reported that macrophages expressing M1 transcription factors (pSTAT1/RBP-J) also frequently express CD163. Indeed, in TH1-polarised conditions characterised by M1 macrophage expansion, CD163-expressing cells remain the most frequent. CD163 is expressed in lesions of severe IgA nephropathy, membranoproliferative glomerulonephritis and lupus nephritis. A recent study in lupus nephritis demonstrated that >70% of CD68+ glomerular macrophages were CD163+, and that urinary CD163 levels correlated with glomerular CD163 expression. Urine sCD163 levels have also been associated with identifying AKI in patients with sepsis, but the values were not significant (Su et al. BMC Nephrology, 2012, 13:123). CD163 is not expressed in normal human glomeruli, non-inflammatory kidney disease (such as myeloma kidney) and expression is limited to the tubulointerstitial space in interstitial nephritis. The oligonucleotide sequence of the sCD163 gene (SEQ ID NO: 1), mRNA encoding the protein (SEQ ID NOs: 2, 3, 4 and 5 - variants) and the protein (SEQ ID NO: 6) are provided below. It should be understood that any one or all of SEQ ID NOs: 1 to 6 may be detected in the sample obtained from a patient.

SEQ ID NO: 1 - DNA: NC_000012.12 (7470816..7503818)
atatgtagcc ttttcatttt catgaaagtg aagtgatttt tagaattctt agttgttttc
tttagaagaa catttctagg gaataataca agaagattta ggaatcattg aagttataaa
tctttggaat gagcaaactc agaatggtgc tacttgaaga ctctggatct gctgacttca
gaagacattt tgtcaacttg agtcccttca ccattactgt ggtcttactt ctcagtgcct
gttttgtcac cagttctctt ggaggaacag acaaggagct gaggctagtg gatggtgaaa
acaagtgtag cgggagagtg gaagtgaaag tccaggagga gtggggaacg gtgtgtaata
atggctggag catggaagcg gtctctgtga tttgtaacca gctgggatgt ccaactgcta
tcaaagcccc tggatgggct aattccagtg caggttctgg acgcatttgg atggatcatg
tttcttgtcg tgggaatgag tcagctcttt gggattgcaa acatgatgga tggggaaagc
atagtaactg tactcaccaa caagatgctg gagtgacctg ctcagatgga tccaatttgg
aaatgaggct gacgcgtgga gggaatatgt gttctggaag aatagagatc aaattccaag
gacggtgggg aacagtgtgt gatgataact tcaacataga tcatgcatct gtcatttgta
gacaacttga atgtggaagt gctgtcagtt tctctggttc atctaatttt ggagaaggct
ctggaccaat ctggtttgat gatcttatat gcaacggaaa tgagtcagct ctctggaact
gcaaacatca aggatgggga aagcataact gtgatcatgc tgaggatgct ggagtgattt
gctcaaaggg agcagatctg agcctgagac tggtagatgg agtcactgaa tgttcaggaa
gattagaagt gagattccaa ggagaatggg ggacaatatg tgatgacggc tgggacagtt
acgatgctgc tgtggcatgc aagcaactgg gatgtccaac tgccgtcaca gccattggtc
gagttaacgc cagtaaggga tttggacaca tctggcttga cagcgtttct tgccagggac
atgaacctgc tatctggcaa tgtaaacacc atgaatgggg aaagcattat tgcaatcaca
atgaagatgc tggcgtgaca tgttctgatg gatcagatct ggagctaaga cttagaggtg
gaggcagccg ctgtgctggg acagttgagg tggagattca gagactgtta gggaaggtgt

gtgacagagg ctggggactg aaagaagctg atgtggtttg caggcagctg ggatgtggat
ctgcactcaa aacatcttat caagtgtact ccaaaatcca ggcaacaaac acatggctgt
ttctaagtag ctgtaacgga aatgaaactt ctctttggga ctgcaagaac tggcaatggg
gtggacttac ctgtgatcac tatgaagaag ccaaaattac ctgctcagcc cacaggaacc
ccagactggt tggaggggac attccctgtt ctggacgtgt tgaagtgaag catggtgaca
cgtggggctc catctgtgat tcggacttct ctctggaagc tgccagcgtt ctatgcaggg
aattacagtg tggcacagtt gtctctatcc tggggggagc tcactttgga gagggaaatg
gacagatctg ggctgaagaa ttccagtgtg agggacatga gtcccatctt tcactctgcc
cagtagcacc ccgcccagaa ggaacttgta gccacagcag ggatgttgga gtagtctgct
caagatacac agaaattcgc ttggtgaatg gcaagacccc gtgtgagggc agagtggagc
tcaaaacgct tggtgcctgg ggatccctct gtaactctca ctgggacata gaagatgccc
atgttctttg ccagcagctt aaatgtggag ttgccctttc taccccagga ggagcacgtt
ttggaaaagg aaatggtcag atctggaggc atatgtttca ctgcactggg actgagcagc
acatgggaga ttgtcctgta actgctctag gtgcttcatt atgtccttca gagcaagtgg
cctctgtaat ctgctcagga aaccagtccc aaacactgtc ctcgtgcaat tcatcgtctt
tgggcccaac aaggcctacc attccagaag aaagtgctgt ggcctgcata gagagtggtc
aacttcgcct ggtaaatgga ggaggtcgct gtgctgggag agtagagatc tatcatgagg
gctcctgggg caccatctgt gatgacagct gggacctgag tgatgcccac gtggtttgca
gacagctggg ctgtggagag gccattaatg ccactggttc tgctcatttt ggggaaggaa
cagggcccat ctggctggat gagatgaaat gcaatggaaa agaatcccgc atttggcagt
gccattcaca cggctggggg cagcaaaatt gcaggcacaa ggaggatgcg ggagttatct
gctcagaatt catgtctctg agactgacca gtgaagccag cagagaggcc tgtgcagggc
gtctggaagt tttttacaat ggagcttggg gcactgttgg caagagtagc atgtctgaaa
ccactgtggg tgtggtgtgc aggcagctgg gctgtgcaga caaagggaaa atcaaccctg
catctttaga caaggccatg tccattccca tgtgggtgga caatgttcag tgtccaaaag
gacctgacac gctgtggcag tgcccatcat ctccatggga gaagagactg gccagcccct
cggaggagac ctggatcaca tgtgacaaca agataagact tcaggaagga cccacttcct
gttctggacg tgtggagatc tggcatggag gttcctgggg gacagtgtgt gatgactctt
gggacttgga cgatgctcag gtggtgtgtc aacaacttgg ctgtggtcca gctttgaaag
cattcaaaga agcagagttt ggtcagggga ctggaccgat atggctcaat gaagtgaagt
gcaaagggaa tgagtcttcc ttgtgggatt gtcctgccag acgctggggc catagtgagt
gtgggcacaa ggaagacgct gcagtgaatt gcacagatat ttcagtgcag aaaaccccac
aaaaagccac aacaggtcgc tcatcccgtc agtcatcctt tattgcagtc gggatccttg
gggttgttct gttggccatt ttcgtcgcat tattcttctt gactaaaaag cgaagacaga
gacagcggct tgcagtttcc tcaagaggag agaacttagt ccaccaaatt caataccggg
agatgaattc ttgcctgaat gcagatgatc tggacctaat gaattcctca gaaaattccc
atgagtcagc tgatttcagt gctgctgaac taatttctgt gtctaaattt cttcctattt
ctggaatgga aaaggaggcc attctgagcc acactgaaaa ggaaaatggg aatttataac
ccagtgagtt cagcctttaa gataccttga tgaagacctg gactattgaa tggagcagaa
attcacctct ctcactgact attacagttg catttttatg gagttcttct tctcctagga
ttcctaagac tgctgctgaa tttataaaaa ttaagtttgt gaatgtgact acttagtggt
gtatatgaga ctttcaaggg aattaaataa ataaataaga atgttattga tttgagtttg
ctttaattac ttgtccttaa ttctattaat ttctaaatgg gcttcctaat tttttgtaga
gtttcctaga tgtattataa tgtgttttat ttgacagtgt ttcaatttgc atatacagta
ctgtatattt tttcttattt ggtttgaata attttcctat taccaaataa aaataaattt
atttttactt tagtttttct aagacaggaa aagttaatga tattgaaggg tctgtaaata
atatatggct aactttataa ggcatgactc acaacgattc tttaactgct tttgttact
gtaattctgt tcactagaat aaaatgcaga gccacacctg gtgagggcac

SEQ ID NO: 2 - mRNA (NM_004244.5) isoforms: Q86VB7-1 Q86VB7-2 Q86VB7-3
Q86VB7-4
Isoform 1 Q86VB7 amino acid sequence:
MSKLRMVLLEDSGSADFRRHFVNLSPFTITVVLLLSACFVTSSLGGTDKELRLVDGENKCSGRVEVKVQE
EWGTVCNNGWSMEAVSVICNQLGCPTAIKAPGWANSSAGSGRIWMDHVSCRGNESALWDCKHDGWGKHSN
CTHQQDAGVTCSDGSNLEMRLTRGGNMCSGRIEIKFQGRWGTVCDDNFNIDHASVICRQLECGSAVSFSG
SSNFGEGSGPIWFDDLICNGNESALWNCKHQGWGKHNCDHAEDAGVICSKGADLSLRLVDGVTECSGRLE
VRFQGEWGTICDDGWDSYDAAVACKQLGCPTAVTAIGRVNASKGFGHIWLDSVSCQGHEPAIWQCKHHEW
GKHYCNHNEDAGVTCSDGSDLELRLRGGGSRCAGTVEVEIQRLLGKVCDRGWGLKEADVVCRQLGCGSAL
KTSYQVYSKIQATNTWLFLSSCNGNETSLWDCKNWQWGGLTCDHYEEAKITCSAHREPRLVGGDIPCSGR

VEVKHGDTWGSICDSDFSLEAASVLCRELQCGTVVSILGGAHFGEGNGQIWAEEFQCEGHESHLSLCPVA
PRPEGTCSHSRDVGVVCSRYTEIRLVNGKTPCEGRVELKTLGAWGSLCNSHWDIEDAHVLCQQLKCGVAL
STPGGARFGKGNGQIWRHMFHCTGTEQHMGDCPVTALGASLCPSEQVASVICSGNQSQTLSSCNSSSLGP
TRPTIPEESAVACIESGQLRLVNGGGRCAGRVEIYHEGSWGTICDDSWDLSDAHVVCRQLGCGEAINATG
SAHFGEGTGPIWLDEMKCNGKESRIWQCHSHGWGQQNCRHKEDAGVICSEFMSLRLTSEASREACAGRLE
VFYNGAWGTVGKSSMSETTVGVVCRQLGCADKGKINPASLDKAMSIPMWVDNVQCPKGPDTLWQCPSSPW
EKRLASPSEETWITCDNKIRLQEGPTSCSGRVEIWHGGSWGTVCDDSWDLDDAQVVCQQLGCGPALKAFK
EAEFGQGTGPIWLNEVKCKGNESSLWDCPARRWGHSECGHKEDAAVNCTDISVQKTPQKATTGRSSRQSS
FIAVGILGVVLLAIFVALFFLTKKRRQRQRLAVSSRGENLVHQIQYREMNSCLNADDLDLMNSSENSHES
ADFSAAELISVSKFLPISGMEKEAILSHTEKENGNL

SEQ ID NO: 3 Isoform 2 (identifier: Q86VB7-2). Also known as: Long tail variant 2

The amino acid sequence of this isoform differs from the canonical sequence as follows:

1115-1156: ENSHESADFS...SHTEKENGNL → GLWVLGGSIA...GSLDAYNGQE

MSKLRMVLLEDSGSADFRRHFVNLSPFTITVVLLLSACFVTSSLGGTDKELRLVDGENKCSGRVEVKVQE
EWGTVCNNGWSMEAVSVICNQLGCPTAIKAPGWANSSAGSGRIWMDHVSCRGNESALWDCKHDGWGKHSN
CTHQQDAGVTCSDGSNLEMRLTRGGNMCSGRIEIKFQGRWGTVCDDNFNIDHASVICRQLECGSAVSFSG
SSNFGEGSGPIWFDDLICNGNESALWNCKHQGWGKHNCDHAEDAGVICSKGADLSLRLVDGVTECSGRLE
VRFQGEWGTICDDGWDSYDAAVACKQLGCPTAVTAIGRVNASKGFGHIWLDSVSCQGHEPAIWQCKHHEW
GKHYCNHNEDAGVTCSDGSDLELRLRGGGSRCAGTVEVEIQRLLGKVCDRGWGLKEADVVCRQLGCGSAL
KTSYQVYSKIQATNTWLFLSSCNGNETSLWDCKNWQWGGLTCDHYEEAKITCSAHREPRLVGGDIPCSGR
VEVKHGDTWGSICDSDFSLEAASVLCRELQCGTVVSILGGAHFGEGNGQIWAEEFQCEGHESHLSLCPVA
PRPEGTCSHSRDVGVVCSRYTEIRLVNGKTPCEGRVELKTLGAWGSLCNSHWDIEDAHVLCQQLKCGVAL
STPGGARFGKGNGQIWRHMFHCTGTEQHMGDCPVTALGASLCPSEQVASVICSGNQSQTLSSCNSSSLGP
TRPTIPEESAVACIESGQLRLVNGGGRCAGRVEIYHEGSWGTICDDSWDLSDAHVVCRQLGCGEAINATG
SAHFGEGTGPIWLDEMKCNGKESRIWQCHSHGWGQQNCRHKEDAGVICSEFMSLRLTSEASREACAGRLE
VFYNGAWGTVGKSSMSETTVGVVCRQLGCADKGKINPASLDKAMSIPMWVDNVQCPKGPDTLWQCPSSPW
EKRLASPSEETWITCDNKIRLQEGPTSCSGRVEIWHGGSWGTVCDDSWDLDDAQVVCQQLGCGPALKAFK
EAEFGQGTGPIWLNEVKCKGNESSLWDCPARRWGHSECGHKEDAAVNCTDISVQKTPQKATTGRSSRQSS
FIAVGILGVVLLAIFVALFFLTKKRRQRQRLAVSSRGENLVHQIQYREMNSCLNADDLDLMNSSGLWVLG
GSIAAAELISVSKFLPISGMEKEAILGSLDAYNGQE

SEQ ID NO: 4 Isoform 3 (identifier: Q86VB7-3). Also known as: Short tail variant

The amino acid sequence of this isoform differs from the canonical sequence as follows:

1115-1156: ENSHESADFSAAELISVSKFLPISGMEKEAILSHTEKENGNL → GGHSEPH

MSKLRMVLLEDSGSADFRRHFVNLSPFTITVVLLLSACFVTSSLGGTDKELRLVDGENKCSGRVEVKVQE
EWGTVCNNGWSMEAVSVICNQLGCPTAIKAPGWANSSAGSGRIWMDHVSCRGNESALWDCKHDGWGKHSN
CTHQQDAGVTCSDGSNLEMRLTRGGNMCSGRIEIKFQGRWGTVCDDNFNIDHASVICRQLECGSAVSFSG
SSNFGEGSGPIWFDDLICNGNESALWNCKHQGWGKHNCDHAEDAGVICSKGADLSLRLVDGVTECSGRLE
VRFQGEWGTICDDGWDSYDAAVACKQLGCPTAVTAIGRVNASKGFGHIWLDSVSCQGHEPAIWQCKHHEW
GKHYCNHNEDAGVTCSDGSDLELRLRGGGSRCAGTVEVEIQRLLGKVCDRGWGLKEADVVCRQLGCGSAL
KTSYQVYSKIQATNTWLFLSSCNGNETSLWDCKNWQWGGLTCDHYEEAKITCSAHREPRLVGGDIPCSGR
VEVKHGDTWGSICDSDFSLEAASVLCRELQCGTVVSILGGAHFGEGNGQIWAEEFQCEGHESHLSLCPVA
PRPEGTCSHSRDVGVVCSRYTEIRLVNGKTPCEGRVELKTLGAWGSLCNSHWDIEDAHVLCQQLKCGVAL
STPGGARFGKGNGQIWRHMFHCTGTEQHMGDCPVTALGASLCPSEQVASVICSGNQSQTLSSCNSSSLGP
TRPTIPEESAVACIESGQLRLVNGGGRCAGRVEIYHEGSWGTICDDSWDLSDAHVVCRQLGCGEAINATG
SAHFGEGTGPIWLDEMKCNGKESRIWQCHSHGWGQQNCRHKEDAGVICSEFMSLRLTSEASREACAGRLE
VFYNGAWGTVGKSSMSETTVGVVCRQLGCADKGKINPASLDKAMSIPMWVDNVQCPKGPDTLWQCPSSPW
EKRLASPSEETWITCDNKIRLQEGPTSCSGRVEIWHGGSWGTVCDDSWDLDDAQVVCQQLGCGPALKAFK
EAEFGQGTGPIWLNEVKCKGNESSLWDCPARRWGHSECGHKEDAAVNCTDISVQKTPQKATTGRSSRQSS
FIAVGILGVVLLAIFVALFFLTKKRRQRQRLAVSSRGENLVHQIQYREMNSCLNADDLDLMNSSGGHSEP
H

SEQ ID NO: 5 Isoform 4 (identifier: Q86VB7-4)

The amino acid sequence of this isoform differs from the canonical sequence as follows:

579-579: R → SKTQKTSLIGSYTVKGTGLGSHSCLFLKPCLLPG
1115-1156: ENSHESADFSAAELISVSKFLPISGMEKEAILSHTEKENGNL → GGHSEPH

MSKLRMVLLEDSGSADFRRHFVNLSPFTITVVLLLSACFVTSSLGGTDKELRLVDGENKCSGRVEVKVQE
EWGTVCNNGWSMEAVSVICNQLGCPTAIKAPGWANSSAGSGRIWMDHVSCRGNESALWDCKHDGWGKHSN
CTHQQDAGVTCSDGSNLEMRLTRGGNMCSGRIEIKFQGRWGTVCDDNFNIDHASVICRQLECGSAVSFSG
SSNFGEGSGPIWFDDLICNGNESALWNCKHQGWGKHNCDHAEDAGVICSKGADLSLRLVDGVTECSGRLE
VRFQGEWGTICDDGWDSYDAAVACKQLGCPTAVTAIGRVNASKGFGHIWLDSVSCQGHEPAIWQCKHHEW
GKHYCNHNEDAGVTCSDGSDLELRLRGGGSRCAGTVEVEIQRLLGKVCDRGWGLKEADVVCRQLGCGSAL
KTSYQVYSKIQATNTWLFLSSCNGNETSLWDCKNWQWGGLTCDHYEEAKITCSAHREPRLVGGDIPCSGR
VEVKHGDTWGSICDSDFSLEAASVLCRELQCGTVVSILGGAHFGEGNGQIWAEEFQCEGHESHLSLCPVA
PRPEGTCSHSRDVGVVCSSKTQKTSLIGSYTVKGTGLGSHSCLFLKPCLLPGYTEIRLVNGKTPCEGRVE
LKTLGAWGSLCNSHWDIEDAHVLCQQLKCGVALSTPGGARFGKGNGQIWRHMFHCTGTEQHMGDCPVTAL
GASLCPSEQVASVICSGNQSQTLSSCNSSSLGPTRPTIPEESAVACIESGQLRLVNGGGRCAGRVEIYHE
GSWGTICDDSWDLSDAHVVCRQLGCGEAINATGSAHFGEGTGPIWLDEMKCNGKESRIWQCHSHGWGQQN
CRHKEDAGVICSEFMSLRLTSEASREACAGRLEVFYNGAWGTVGKSSMSETTVGVVCRQLGCADKGKINP
ASLDKAMSIPMWVDNVQCPKGPDTLWQCPSSPWEKRLASPSEETWITCDNKIRLQEGPTSCSGRVEIWHG
GSWGTVCDDSWDLDDAQVVCQQLGCGPALKAFKEAEFGQGTGPIWLNEVKCKGNESSLWDCPARRWGHSE
CGHKEDAAVNCTDISVQKTPQKATTGRSSRQSSFIAVGILGVVLLAIFVALFFLTKKRRQRQRLAVSSRG
ENLVHQIQYREMNSCLNADDLDLMNSSGGHSEPH

SEQ ID NO: 6 - Protein: NP_004235.4:
MSKLRMVLLEDSGSADFRRHFVNLSPFTITVVLLLSACFVTSSLGGTDKELRLVDGENKCSGRVEVKVQE
EWGTVCNNGWSMEAVSVICNQLGCPTAIKAPGWANSSAGSGRIWMDHVSCRGNESALWDCKHDGWGKHSN
CTHQQDAGVTCSDGSNLEMRLTRGGNMCSGRIEIKFQGRWGTVCDDNFNIDHASVICRQLECGSAVSFSG
SSNFGEGSGPIWFDDLICNGNESALWNCKHQGWGKHNCDHAEDAGVICSKGADLSLRLVDGVTECSGRLE
VRFQGEWGTICDDGWDSYDAAVACKQLGCPTAVTAIGRVNASKGFGHIWLDSVSCQGHEPAIWQCKHHEW
GKHYCNHNEDAGVTCSDGSDLELRLRGGGSRCAGTVEVEIQRLLGKVCDRGWGLKEADVVCRQLGCGSAL
KTSYQVYSKIQATNTWLFLSSCNGNETSLWDCKNWQWGGLTCDHYEEAKITCSAHREPRLVGGDIPCSGR
VEVKHGDTWGSICDSDFSLEAASVLCRELQCGTVVSILGGAHFGEGNGQIWAEEFQCEGHESHLSLCPVA
PRPEGTCSHSRDVGVVCSRYTEIRLVNGKTPCEGRVELKTLGAWGSLCNSHWDIEDAHVLCQQLKCGVAL
STPGGARFGKGNGQIWRHMFHCTGTEQHMGDCPVTALGASLCPSEQVASVICSGNQSQTLSSCNSSSLGP
TRPTIPEESAVACIESGQLRLVNGGGRCAGRVEIYHEGSWGTICDDSWDLSDAHVVCRQLGCGEAINATG
SAHFGEGTGPIWLDEMKCNGKESRIWQCHSHGWGQQNCRHKEDAGVICSEFMSLRLTSEASREACAGRLE
VFYNGAWGTVGKSSMSETTVGVVCRQLGCADKGKINPASLDKAMSIPMWVDNVQCPKGPDTLWQCPSSPW
EKRLASPSEETWITCDNKIRLQEGPTSCSGRVEIWHGGSWGTVCDDSWDLDDAQVVCQQLGCGPALKAFK
EAEFGQGTGPIWLNEVKCKGNESSLWDCPARRWGHSECGHKEDAAVNCTDISVQKTPQKATTGRSSRQSS
FIAVGILGVVLLAIFVALFFLTKKRRQRQRLAVSSRGENLVHQIQYREMNSCLNADDLDLMNSSENSHES
ADFSAAELISVSKFLPISGMEKEAILSHTEKENGNL

In the specification, the term Monocyte chemotactic protein-1 (MCP-1) should be understood to be a member of the small inducible gene (SIG) family, which plays a role in the recruitment of monocytes to sites of injury and infection. The gene for MCP-1 is on chromosome 17 in region 17q11.2-q12. MCP-1 has been found in the joints of people with rheumatoid arthritis where may serve to recruit macrophages and perpetuate the inflammation in the joints. MPC-1 has also been found elevated in the urine of people with lupus.

In the specification, the term "reference sample" or "reference sample level" as applied to sCD163 and MCP-1 should be understood to mean a reference level of sCD163 and/or MCP-1 detected in a sample obtained from an individual identified as having kidney disease, where the kidney disease is *not* due to SVV, CGN mimics, acute active CGN or other forms of CGN.

The methods of the invention are applicable to individuals who are healthy (not having established kidney disease), as well as individuals with established CGN, individuals at risk of having CGN or individuals deemed to be at risk of having a recurrence of CGN (CGN flare).

The term "biological sample" refers to any biological material from the individual for example blood, serum, urine, saliva, tissue, cerebrospinal fluid and the like. Typically, the biological sample is, or is obtained from, urine.

In this specification, the terms "treating", "treatment" or "induction treatment" refers to administering a medicament, for example one or more glucocorticoid or immunosuppressant, to an individual who has, or is likely to have, CGN or CGN flare with the purpose to induce remission of the disease or to cure, heal, prevent, alleviate, relieve, alter, remedy, ameliorate, or improve the disease. The term "therapeutically effective amount" refers to the amount of the medicament, for example a glucocorticoid, that is required to confer the intended therapeutic effect in the individual, which amount will vary depending on the type of medicament, route of administration, type and cause of the CGN/CGN flare, and possible inclusion of other therapeutics or excipients. The terms can be used interchangeably.

To practice one or more methods of this invention, the above-described pharmaceutical composition (medicament) can be administered orally, parenterally, by inhalation spray, topically, rectally, nasally, bucally, vaginally or via an implanted reservoir. The term "parenteral" as used herein includes subcutaneous, intracutaneous, intravenous, intramuscular, intraarticular, intraarterial, intrasynovial, intrasternal, intrathecal, intralesional, and intracranial injection or infusion techniques. A sterile injectable composition, e.g., a sterile injectable aqueous or oleaginous suspension, can be formulated according to techniques known in the art using suitable dispersing or wetting agents (such as Tween 80) and suspending agents. The sterile injectable preparation can also be a sterile injectable solution or suspension in a non-toxic parentally acceptable diluents or solvent for example, as a solution in 1,3-butanediol. Among the acceptable vehicles and solvents that can be employed are mannitol, water, Ringer's solution and isotonic sodium chloride solution. In addition, sterile, fixed oils are conventionally employed as a solvent or suspending medium (e.g. Synthetic mono-or dyglycerides). Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions can also contain a long-chain alcohol diluents or dispersant, or carboxymethyl cellulose or similar dispersing agents. Other commonly used surfactants such as Tweens or Spans or other similar emulsifying agents or bioavailablity enhancers which are commonly used in the manufacture of pharmaceutically acceptable solid, liquid, or other dosage forms can also be used for the purposes of formulation.

A composition for oral administration can be any orally acceptable dosage form including, but not limited to, capsules, tablets, emulsions and aqueous suspensions, dispersions and solutions. In the case of tablets for oral use, carriers that are commonly used include lactose and corn starch. Lubricating agents, such as magnesium stearate, are also typically added. For oral administration in a capsule form, useful diluents include lactose and dried corn starch. When aqueous suspensions or emulsions are administered orally, the active ingredient can be suspended or dissolved in an oily phase combined with emulsifying or suspending agents. If desired, certain sweetening, flavouring, or colouring agents can be added. A nasal aerosol or inhalation composition can be prepared according to techniques well known in the art of pharmaceutical formulation. A fused multicyclic compound-containing composition can also be administered in the form of suppositories for rectal administration.

The carrier in the pharmaceutical composition must be "acceptable" in the sense of being compatible with the active ingredient of the formulation (and preferable, capable of stabilising it) and not deleterious to the subject to be treated. For example, one or more solubilising agents, which form more soluble complexes with the fused multicyclic compounds, or more solubilising agents, can be utilised as pharmaceutical carriers for delivery of the active compounds. Examples of other carriers include colloidal silicon dioxide, magnesium stearate, sodium lauryl sulphate, and D&C Yellow #10.

The term "glucocorticoid" should be understood to mean, in the context of treating small vessel vasculitis and CGN, a synthetic corticosteroid designed to act as an antiinflammatory agent. Typical glucorticoids include methylprednisolone, prednisolone (known as prednisone in North America), dexamethasone and hydrocortisone.

The term "immunosuppressant" should be understood to mean, in the context of treating small vessel vasculitis and CGN, an agent designed to suppress or eliminate activated immune cells. Typical immunosuppressants include cyclophosphamide, rituximab, azathioprine, mycophenolate mofetil and methotrexate.

sCD163-specific antibodies and MCP-1 specific antibodies may be produced using methods which are generally known in the art. In particular, purified sCD163 (and MCP-1) may be used to produce antibodies or to screen libraries of pharmaceutical agents to identify those which specifically bind sCD163. Antibodies to sCD163 and antibodies to MCP-1 may also be generated using methods that are well known in the art. Such antibodies may include, but are not limited to, polyclonal, monoclonal, chimeric, and single chain antibodies, Fab fragments, and fragments produced by a Fab expression library. Single chain antibodies (e.g., from camels or llamas) may have advantages in the development of immuno-adsorbents and biosensors (Muyldermans, S. (2001) J. Biotechnol. 74:277-302). For the production of antibodies, various hosts including goats, rabbits, rats, mice, camels, dromedaries, llamas, humans, and others may be immunized by injection with sCD163 or with any fragment or oligopeptide thereof (or injection with MCP-1 or with any fragment or oligopeptide thereof) which has immunogenic properties. Depending on the host species, various adjuvants may be used to increase immunological response. Such adjuvants include, but are not limited to, Freund's, mineral gels such as aluminum hydroxide, and surface active substances such as lysolecithin, pluronic polyols, polyanions, peptides, oil emulsions, KLH, and dinitrophenol.

It is preferred that the oligopeptides, peptides, or fragments used to induce antibodies to sCD163 and to MCP-1 have an amino acid sequence consisting of at least about 5 amino acids, and generally will consist of at least about 10 amino acids. It is also preferable that these oligopeptides, peptides, or fragments are identical to a portion of the amino acid sequence of the natural protein. Short stretches of sCD163 and MCP-1 amino acids may be fused with those of another protein, such as keyhole limpet hemocyanin (KLH), and antibodies to the chimeric molecule may be produced. Monoclonal antibodies to sCD163 and to MCP-1 may be prepared using any technique which provides for the production of antibody molecules by continuous cell lines in culture. These include, but are not limited to, the hybridoma technique, the human B-cell hybridoma technique, and the EBV-hybridoma technique. (See, e.g., Kohler, G. et al. (1975) Nature 256:495-497; Kozbor, D. et al. (1985) J. Immunol. Methods 81:31-42; Cote, R. J. et al. (1983) Proc. Natl. Acad. Sci. USA 80:2026-2030; and Cole, S.P. et al. (1984) Mol. Cell. Biol. 62:109-120.)

In addition, techniques developed for the production of "chimeric antibodies", such as the splicing of mouse antibody genes to human antibody genes to obtain a molecule with appropriate antigen specificity and biological activity, can be used (see, e.g., Morrison, S. L. et al. (1984) Proc. Natl. Acad. Sci. USA 81:6851-6855; Neuberger, M. S. et al. (1984) Nature 312:604-608; and Takeda, S. et al. (1985) Nature 314:452-454). Alternatively, techniques described for the production of single chain antibodies may be adapted, using methods known in the art, to produce sCD163-specific or MCP-1-specific single chain antibodies. Antibodies with related specificity, but of distinct idiotypic composition, may be generated by chain shuffling from random combinatorial immunoglobulin libraries (see, *e.g.,* Burton, D. R. (1991) Proc. Natl. Acad. Sci. USA 88:10134-10137.). Antibodies may also be produced by inducing *in vivo* production in the lymphocyte population or by screening immunoglobulin libraries or panels of highly specific binding reagents as disclosed in the literature (see, *e.g.,* Orlandi, R. et al. (1989) Proc. Natl. Acad. Sci. USA 86:3833-3837; Winter, G. et al. (1991) Nature 349:293-299).

Antibody fragments which contain specific binding sites for sCD163 and specific binding sites for MCP-1 may also be generated. For example, such fragments include, but are not limited to, F(ab')2 fragments produced by pepsin digestion of the antibody molecule and Fab fragments generated by reducing the disulfide bridges of the F(ab')2 fragments.

Alternatively, Fab expression libraries may be constructed to allow rapid and easy identification of monoclonal Fab fragments with the desired specificity (see, *e.g.,* Huse, W. D. et al. (1989) Science 246:1275-1281).

Various immunoassays may be used for screening to identify antibodies having the desired specificity. Numerous protocols for competitive binding or immunoradiometric assays using either polyclonal or monoclonal antibodies with established specificities are well known in the art. Such immunoassays typically involve the measurement of complex formation between sCD163 and its specific antibody, and also formation between MCP-1 and its specific antibody. A two-site, monoclonal-based immunoassay utilizing monoclonal antibodies reactive to two non-interfering sCD163 epitopes is generally used, but a competitive binding assay may also be employed. Various methods such as Scatchard analysis in conjunction with radioimmunoassay techniques may be used to assess the affinity of antibodies for sCD163 and for MCP-1. Affinity is expressed as an association constant, Ka, which is defined as the molar concentration of sCD163-antibody complex divided by the molar concentrations of free antigen and free antibody under equilibrium conditions. Similar analysis can be done for the MCP-1-antibody complex. The Ka determined for a preparation of polyclonal antibodies, which are heterogeneous in their affinities for multiple sCD163 (or MCP-1) epitopes, represents the average affinity, or avidity, of the antibodies for sCD163 (or MCP-1). The Ka determined for a preparation of monoclonal antibodies, which are monospecific for a particular sCD163 (or MCP-1) epitope, represents a true measure of affinity. High-affinity antibody preparations with Ka ranging from about 10⁹ to 10¹² L/mole are preferred for use in immunoassays in which the sCD163-antibody complex (or MCP-1-antibody complex) must withstand rigorous manipulations.

The titre and avidity of polyclonal antibody preparations may be further evaluated to determine the quality and suitability of such preparations for certain downstream applications. For example, a polyclonal antibody preparation containing at least 1-2 mg specific antibody/ml, preferably 5-10 mg specific antibody/ml, is generally employed in procedures requiring precipitation of sCD163-antibody complexes (or MCP-1-anitbody complexes). Procedures for evaluating antibody specificity, titre and avidity, and guidelines for antibody quality and usage in various applications, are generally available.

Embodiments of the invention can be described through functional modules, which are defined by computer executable instructions recorded on computer readable media and which cause a computer to perform method steps when executed. The modules are segregated by function for the sake of clarity. However, it should be understood that the modules/systems need not correspond to discreet blocks of code and the described functions can be carried out by the execution of various code portions stored on various media and executed at various times. Furthermore, it should be appreciated that the modules may perform other functions, thus the modules are not limited to having any particular functions or set of functions.

The computer readable storage media #30 can be any available tangible media that can be accessed by a computer. Computer readable storage media includes volatile and nonvolatile, removable and non-removable tangible media implemented in any method or technology for storage of information such as computer readable instructions, data structures, program modules or other data. Computer readable storage media includes, but is not limited to, RAM (random access memory), ROM (read only memory), EPROM (erasable programmable read only memory), EEPROM (electrically erasable programmable read only memory), flash memory or other memory technology, CD-ROM (compact disc read only memory), DVDs (digital versatile disks) or other optical storage media, magnetic cassettes, magnetic tape, magnetic disk storage or other magnetic storage media, other types of volatile and non-volatile memory, and any other tangible medium which can be used to store the desired information and which can accessed by a computer including and any suitable combination of the foregoing.

Computer-readable data embodied on one or more computer-readable storage media may define instructions, for example, as part of one or more programs that, as a result of being executed by a computer, instruct the computer to perform one or more of the functions described herein, and/or various embodiments, variations and combinations thereof. Such instructions may be written in any of a plurality of programming languages, for example, Java, J#, Visual Basic, C, C#, C++, Fortran, Pascal, Eiffel, Basic, COBOL assembly language, and the like, or any of a variety of combinations thereof. The computer-readable storage media on which such instructions are embodied may reside on one or more of the components of either of a system, or a computer readable storage medium described herein, may be distributed across one or more of such components.

The computer-readable storage media may be transportable such that the instructions stored thereon can be loaded onto any computer resource to implement the aspects of the present invention discussed herein. In addition, it should be appreciated that the instructions stored on the computer-readable medium, described above, are not limited to instructions embodied as part of an application program running on a host computer. Rather, the instructions may be embodied as any type of computer code (e.g., software or microcode) that can be employed to program a computer to implement aspects of the present invention. The computer executable instructions may be written in a suitable computer language or combination of several languages. Basic computational biology methods are known to those of ordinary skill in the art and are described in, for example, Setubal and Meidanis et al., Introduction to Computational Biology Methods (PWS Publishing Company, Boston, 1997); Salzberg, Searles, Kasif, (Ed.), Computational Methods in Molecular Biology, (Elsevier, Amsterdam, 1998); Rashidi and Buehler, Bioinformatics Basics: Application in Biological Science and Medicine (CRC Press, London, 2000) and Ouelette and Bzevanis Bioinformatics: A Practical Guide for Analysis of Gene and Proteins (Wiley & Sons, Inc., 2nd ed., 2001).

The functional modules of certain embodiments of the invention include at minimum a determination system #40, a storage device #30, a comparison module #80, and a display module #110. The functional modules can be executed on one, or multiple, computers, or by using one, or multiple, computer networks. The determination system has computer executable instructions to provide e.g., protein, DNA or RNA levels in computer readable form.

The determination system #40, can comprise any system for detecting the levels of sCD163 levels (or MCP-1 levels) when compared to levels in a reference sample. Standard procedures such as an ELISA, RT-PCR, *etc.* can be used.

The information determined in the determination system can be read by the storage device #30. As used herein the "storage device" is intended to include any suitable computing or processing apparatus or other device configured or adapted for storing data or information. Examples of an electronic apparatus suitable for use with the present invention include a stand-alone computing apparatus, data telecommunications networks, including local area networks (LAN), wide area networks (WAN), Internet, Intranet, and Extranet, and local and distributed computer processing systems. Storage devices also include, but are not limited to: magnetic storage media, such as floppy discs, hard disc storage media, magnetic tape, optical storage media such as CD-ROM, DVD, electronic storage media such as RAM, ROM, EPROM, EEPROM and the like, general hard disks and hybrids of these categories such as magnetic/optical storage media. The storage device is adapted or configured for having recorded thereon nucleic acid sequence information. Such information may be provided in digital form that can be transmitted and read electronically, e.g., via the Internet, on diskette, via USB (universal serial bus) or via any other suitable mode of communication.

As used herein, "stored" refers to a process for encoding information on the storage device. Those skilled in the art can readily adopt any of the presently known methods for recording information on known media to generate manufactures comprising information relating to levels of sCD163 (and/or MCP-1).

In one embodiment the reference data stored in the storage device to be read by the comparison module is compared, e.g., detection of levels of sCD163 (and/or MCP-1) in a sample and correlates with recurrence of CGN or suffering with CGN.

The "comparison module" #80 can use a variety of available software programs and formats for the comparison operative to compare levels of sCD163 data determined in the determination system to reference samples and/or stored reference data. In one embodiment, the comparison module is configured to use pattern recognition techniques to compare information from one or more entries to one or more reference data patterns. The comparison module may be configured using existing commercially-available or freely-available software for comparing patterns, and may be optimized for particular data comparisons that are conducted. The comparison module provides computer readable information related to the levels of sCD163 (and/or MCP-1) in the sample.

The comparison module, or any other module of the invention, may include an operating system (e.g., UNIX) on which runs a relational database management system, a World Wide Web application, and a World Wide Web server. World Wide Web application includes the executable code necessary for generation of database language statements (e.g., Structured Query Language (SQL) statements). Generally, the executables will include embedded SQL statements. In addition, the World Wide Web application may include a configuration file which contains pointers and addresses to the various software entities that comprise the server as well as the various external and internal databases which must be accessed to service user requests. The Configuration file also directs requests for server resources to the appropriate hardware--as may be necessary should the server be distributed over two or more separate computers. In one embodiment, the World Wide Web server supports a TCP/IP protocol. Local networks such as this are sometimes referred to as "Intranets." An advantage of such Intranets is that they allow easy communication with public domain databases residing on the World Wide Web (*e.g.,* the GenBank or Swiss Pro World Wide Web site). Thus, in a particular preferred embodiment of the present invention, users can directly access data (via Hypertext links for example) residing on Internet databases using a HTML interface provided by Web browsers and Web servers.

The comparison module provides a computer readable comparison result that can be processed in computer readable form by predefined criteria, or criteria defined by a user, to provide a content based in part on the comparison result that may be stored and output as requested by a user using a display module #110.

In one embodiment of the invention, the content based on the comparison result is displayed on a computer monitor #120. In one embodiment of the invention, the content based on the comparison result is displayed through printable media #130, #140. The display module can be any suitable device configured to receive from a computer and display computer readable information to a user. Non-limiting examples include, for example, general-purpose computers such as those based on Intel PENTIUM-type processor, Motorola PowerPC, Sun UltraSPARC, Hewlett-Packard PA-RISC processors, any of a variety of processors available from Advanced Micro Devices (AMD) of Sunnyvale, California, or any other type of processor, visual display devices such as flat panel displays, cathode ray tubes and the like, as well as computer printers of various types.

In one embodiment, a World Wide Web browser is used for providing a user interface for display of the content based on the comparison result. It should be understood that other modules of the invention can be adapted to have a web browser interface. Through the Web browser, a user may construct requests for retrieving data from the comparison module. Thus, the user will typically point and click to user interface elements such as buttons, pull down menus, scroll bars and the like conventionally employed in graphical user interfaces.

The methods described herein therefore provide for systems (and computer readable media for causing computer systems) to perform methods as described in the Statements of Invention above, for example (a) methods of identifying an individual with active CGN or experiencing a CGN flare, (b) methods of identifying an individual most likely to gain benefit from continuance of treatment for CGN (assessing response to induction treatment), (c) methods of identifying an individual most likely to gain benefit from cessation of treatment for CGN or a reduced level of treatment (assessing response to induction treatment), (d) methods of distinguishing CGN flare from other causes of clinical decline in an individual known previously to have CGN or a systemic autoimmune disease typically associated with CGN (distinguishing small vessel vasculitis from CGN mimics), and (e) methods for treating an individual with established CGN or recurrence of CGN, and treating said individual with glucocorticoid and/or immunosuppressant therapy according the appended claims.

Systems and computer readable media described herein are merely illustrative embodiments of the invention for performing methods of diagnosis in an individual. Variations of the systems and computer readable media described herein are possible.

The modules of the machine, or those used in the computer readable medium, may assume numerous configurations. For example, function may be provided on a single machine or distributed over multiple machines.

Methods for assaying a biological sample for the presence of one or more biomarkers will be well known to those skilled in the art, for example, by immunodetection. In this specification, the term "immunodetection" should be understood to mean an antibody labelled to facilitate detection. That is, where another molecule is incorporated in the antibody, for example, incorporation of a radiolabelled amino acid. Various methods of labelling are known in the art and may be used, for example, radioisotopes or radionuclides (e.g., 3H, 14C, 15N, 35S), fluorescent labels (e.g., FITC, rhodamine, lanthanide phosphors), enzymatic labels (e.g., horseradish peroxidase, beta-galactosidase, luciferase, alkaline phosphatase), chemiluminescent markers, biotinyl groups, predetermined polypeptide epitopes recognized by a secondary reporter (e.g., leucine zipper pair sequences, binding sites for secondary antibodies, metal binding domains, epitope tags), magnetic agents, such as gadolinium chelates, and toxins such as pertussis toxin, ethidium bromide, etoposide, vincristine, colchicin, doxorubicin, daunorubicin, dihydroxy anthracin dione, mitoxantrone, mithramycin, actinomycin D, 1-dehydrotestosterone, and analogs or homologs thereof. Such immunodetection assays or immunoassays include, but are not limited to, immunoprecipitation assays, Enzyme-linked Immunosorbent Assay (ELISA)-based assays, dip-stick assays, immunofluorescence microscopy, magnetic immunoassay, radioimmunoassay, "sandwich" immunoassays, immunodiffusion assays, agglutination assays, and Western blot assays.

Other methods of detecting biomarkers will be well known to those skilled in the field of proteomics. For example, protein arrays may be generated that are custom-made to assay for the presence of specific auto-antibodies. The company RZPD (www.rzpd.de) provide a service for the production of customised macro-arrays. Other methods for the detection of the biomarkers of the invention would be the use of ELISA's, the details of which will be well known to those skilled in the field.

Other methods of detecting the biomarkers that are well known to those skilled in the art are those that detect the oligonucleotides encoding the biomarkers of interest, for example, Polymerase Chain Reaction (PCR) method, Reverse Transcriptase PCR (RT-PCR), Southern and Northern Blotting, Fluorescence in-situ hybridisation and nucleic acid hybridisation to micro-array.

The proteins used in these techniques can be prepared using standard recombinant DNA techniques along with the sequence information provided herein and in the cross-referenced NCBI database (see Sequence Listing). In the case of many of the proteins of SEQUENCE ID NO's 1 to 6, bacterial clones engineered to express the protein of interest are available from various commercial sources.

In the specification, the term "correlating device" should be understood to mean an apparatus that is capable of correlating the detectable signal with an elevated level of sCD163, and optionally MCP-1. In one embodiment, the device comprises a comparative reference module (or correlating device), such as a colour chart, used in an *in vitro* diagnostic apparatus, such as a dipstick assay or an ELISA, to determine an elevated level of sCD163, and optionally MCP-1, in a biological sample. In another embodiment, the correlating device may comprise a detection module configured for detection of the detectable signal, a comparison module configured to compare the detectable signal with a reference signal and provide an output relating to whether the level of sCD163, and optionally MCP-1, is elevated, and a display module for displaying the output.

### Materials and Methods

### Induction of experimental autoimmune vasculitis (EAV)

Experimental autoimmune vasculitis, a model of MPO ANCA vasculitis, was induced in rats as previously describedl3, Briefly, 6 week old Wistar-Kyoto (WKY) rats were immunised with 3.2mg/kg human MPO (kind gift from Biovitrum, Sweden) in complete Freund's adjuvant (CFA) along with 1µg of Pertussis toxin (Sigma Aldrich). Rats were boosted with 4µg LPS and 0.1mg/kg MPO (without adjuvant) after 28 and 35 days respectively. Control animals were left un-immunised. Urine samples were collected at several time points during EAV progression by placing rats in metabolic cages for 24 hours. Urine samples were centrifuged at 2000g for 10 minutes at 4°C and stored at -80°C until analysis. sCD163 levels were measured in neat urine using a rat sCD163/soluble haemoglobin scavenger receptor kit (BlueGene), following the manufacturer's instructions. Rats were sacrificed at day 28 and 56 post immunisation and renal sections were stained with haemotoxylin and eosin and periodic acid Schiff stains. Animal studies were approved by local ethics committee and complied with a project license granted by the Irish Medicines Board.

### Immunohistochemistry of EAV renal tissue

Kidneys harvested from rats 56 days after EAV induction were sectioned and blocked with 20% normal goat serum. Sections were dual stained with antibodies specific for rat CD68 (rabbit polyclonal, Abcam) and rat CD 163 (mouse anti-rat ED2, AbD Serotec, used for all rat CD163 staining) followed by alexafluor 568 goat anti-mouse IgG (Life Technologies) and alexafluor 488 goat anti-rabbit IgG (Abcam). Sections were also dual stained with antibodies specific for rat CCR7 (rabbit monoclonal, Abcam) or rat CD206 (rabbit polyclonal, Abcam) and rat CD163, followed by alexafluor goat anti-rabbit IgG 488 and alexafluor goat anti-mouse IgG 568, as above. Nuclei were stained with Hoechst 33342 (Life Technologies). The number of CD68+ and CD163+ cells per glomerulus were scored blindly for at least 30 glomeruli and ten 40X fields (without glomeruli) using a fluorescent microscope (Nikon Eclipse 90i). Images were obtained using a confocal microscope (Zeiss LSM 510).

### Clinical recruitment and assessment

Patients with SVV (comprising granulomatosis with polyangiitis, microscopic polyangiitis, eosinophilic granulomatosis with polyangiitis and anti-GBM disease), healthy controls and disease controls were recruited through the Rare Kidney Disease (RKD) Biobank. Disease controls (Table 1) were divided into those with glomerulonephritis in whom glomerular macrophage infiltration may be expected, those without glomerulonephritis and intensive care unit patients with non-immune mediated acute kidney injury, multi-system failure and sepsis. To address the latter, we tested a large number of urine samples from the Prospective Validation of Acute Kidney Injury Biomarkers and Definitions in Critically ill Patients study. Samples were archived until analysis in the RKD Biobank and the linked registry was used to define clinical details. The study was approved by the institutional review board (reference 2012/38/04) and all recruits provided written, informed consent. All vasculitis patients were classified according to the Chapel Hill consensus classification criteria (Jennette, J.C., et al., 2012 revised international Chapel Hill consensus conference nomenclature of vasculitides. Arthritis Rheum, 2013. 65(1): p. 1-11). The healthy control group were self-reported as healthy and had a normal urine dipstick examination. Vasculitis disease activity was recorded using the Birmingham Vasculitis Activity Score (BVAS) (Stone, J.H., et al., A disease-specific activity index for Wegener's granulomatosis: modification of the Birmingham Vasculitis Activity Score. International Network for the Study of the Systemic Vasculitides (INSSYS). Arthritis Rheum, 2001. 44(4): p. 912-20). Active vasculitis was considered to be present with a BVAS score ≥0. Active renal vasculitis was defined as BVAS>0 with one or more renal items, including the presence of haematuria by urine dipstick; urine samples were not routinely assessed for the presence of casts or dysmorphic red cells. Patients with both newly diagnosed and flaring disease were included in the analysis. The presence or absence of active vasculitis was adjudicated without knowledge of the sCD163 level by reference to the clinical state and response to therapy one month after obtaining the sample. For the purpose of assessment of biomarker performance, we randomly assigned each recruit to either an inception or one of two validation cohorts prior to measurement of sCD163 levels.

**Table 1 Summary of diagnoses in the disease control cohort. *FSGS=Focal and segmental glomerulosclerosis; #AKI = Acute Kidney injury**

| | **N** | **Glomerular macrophage infiltration expected** |
|---|---|---|
| **Non-vasculitic glomerulonephritis** | *30* | |
| *IgA nephropathy* | 9 | Yes |
| *Lupus nephritis* | 2 | Yes |
| *C3 glomerulopathy* | 3 | Yes |
| *Minimal change disease* /*FSGS^{*}* | 6 | No |
| *Cryoglobulinaemia* | 2 | Yes |
| *Others* | 8 | Yes |
| | | |
| **Disease controls without glomerular inflammation** | *54* | |
| *Hypertensive nephrosclerosis* | 12 | No |
| *Diabetic nephropathy* | 11 | No |
| *Interstitial nephritis* | 3 | No |
| *Aortic endovascular stent infection* | 2 | No |
| *Pyelonephritis* | 3 | No |
| *Sjogren's syndrome* | 2 | No |
| *Other primary kidney disease* | 5 | No |
| *Other extra-renal disease* | 16 | No |
| **ITU patients without sepsis or AKI^{#}** | 463 | No |
| **ITU patients with sepsis but no AKI^{#}** | 286 | No |
| **ITU patients with sepsis and AKI^{#}** | 32 | No |

### CD163 staining of human kidney sections

All recruits undergoing a clinically indicated kidney biopsy at one of the study sites (Beaumont Hospital) were further analysed by immunohistochemistry of renal tissue to determine the degree and pattern of CD163 expression. Formalin fixed paraffin wax embedded renal tissue sections were run with the ultraView Universal DAB detection kit run on the Benchmark XT (Roche) automated immuno staining machine. Cell Conditioning 1 (CC1, Roche) was used for 30 mins at 96°C to retrieve the antigen. Mouse anti-human mAb CD163 (10D6, Abcam) and ref ab74604 (Abcam) were used at a dilution of 1/20 for 40 minutes at 37°C. CD163 expression was quantified using the technique of Zhao *et al* (Zhao, L., David, M. Z., Hyjek, E., Chang, A. & Meehan, S. M. M2 macrophage infiltrates in the early stages of ANCA-associated pauci-immune necrotizing GN. Clin J Am Soc Nephrol 10, 54-62, doi:10.2215/CJN.03230314 (2015)). Expression was assessed within areas of fibrinoid necrosis/crescent formation, within normal appearing glomeruli, in the periglomerular region, within tubules and in the interstitial space11. Scores were assigned as follows: 0=no CD163+ cells, 1=1-5 CD163+ cells, 2=6-10 CD163+ cells and 3=more than 10 CD163+ cells. Values were calculated per glomerular cross section for each of the three glomerular regions, and by estimating the fraction of tubular or interstitial compartments with CD163+ cells and multiplying this by the score. To derive a quantitative measure to compare to the urine sCD163 value we performed image analysis on samples with a paired urine sCD163 value using the Visiopharm Integrator System (Visiopharm, Hoersholm, Denmark) (Supplemental Figure 5). The number of nuclei associated with CD163 staining was quantified in glomerular, obsolescent glomerular and extra-glomerular (renal cortex) compartments, the same threshold being applied to all sections.

### Micro dissection and CD163 mRNA quantification in human kidney

Human renal biopsy specimens and Affymetrix microarray expression data were procured within the framework of the European Renal cDNA Bank - Kröner-Fresenius Biopsy Bank (Cohen et al Kidney Int, 61: 133-140, 2002) (see reference (Martini et al JASN 2014, Vol 25, No 11, 2559-2572) for participating centers). Biopsies were obtained from patients after informed consent and with approval of the local ethics committees. Following renal biopsy, the tissue was transferred to RNase inhibitor and microdissected into glomerular and tubular fragments. Total RNA was isolated from microdissected glomeruli, reverse transcribed, and linearly amplified according to a protocol previously reported (Cohen et al Proc Natl Acad Sci U S A, 103: 5682-5687, 2006). The microarray expression data used in this study came from individual patients with diabetic nephropathy (DN), minimal change disease (MCD), IgA nephropathy (IgA), focal segmental glomerulosclerosis (FSGS), membranous nephropathy (MGN), and ANCA-vasculitis (ANCA). Pre-transplantation kidney biopsies from living donors (LD) were used as control renal tissue. Fragmentation, hybridization, staining, and imaging were performed according to the Affymetrix Expression Analysis Technical Manual (Affymetrix, Santa Clara, CA). CEL file normalization was performed with the Robust Multichip Average method using RMAExpress (Version 1.0.5) and the human Entrez-Gene custom CDF annotation from Brain Array version 18 (http://brainarray.mbni.med.umich.edu/Brainarray/default.asp). To identify differentially expressed genes the SAM (Significance analysis of Microarrays) method was applied using TiGR (MeV, Version 4.8.1) (Tusher et al Proc Natl Acad Sci U S A, 98: 5116-5121, 2001). A q-value below 5% was considered to be statistically significant.

### Measurement of sCD163 in human serum and urine samples

Venous blood was collected into serum tubes and allowed to clot at room temperature before being centrifuged at 1,500g for 10 minutes at 4°C. Serum was removed and stored at -80°C until assay. Urine samples were centrifuged at 2,000g for 10 minutes at 4°C, with storage of the supernatant at -80°C until assay. Samples from the Groningen validation cohort were processed slightly different. Venous blood was centrifuged at 2,000g for 10 minutes, after which serum was collected and stored at -20°C until assay. Urine was centrifuged for 15 min at 1,200g, supernatant was collected, diluted 1:1 in PBS and stored at -20°C until assay. Serum and urine sCD163 levels were determined by ELISA (R&D systems Human sCD163 DuoSet, DY1607) following the manufacturer's instructions. Serum and urine samples were diluted 1:400 and 1:4 respectively. Urine sCD163 level were normalised to the creatinine level as determined by a modified Jaffe technique.

### Investigation of the impact of haematuria on urine sCD163 level

To assess whether the urine sCD163 was merely a surrogate marker of haematuria, we obtained urine from a healthy control (confirmed previously as negative for sCD163) and spiked in serial dilutions of anticoagulated blood. At each dilution the urine was tested for blood by dipstick and by visual inspection for macroscopic haematuria. After incubation at room temperature for 1 hour, the urine was centrifuged and sCD163 levels measured as described above.

### Assessment of the impact of prolonged storage and freeze-thaws on sCD163 level

To assess stability of sCD163 under normal urine handling conditions, a series of aliquots from a urine sample were prepared with known elevated sCD163 level and stored at room temperature, 4°C, -20°C and -80°C for up to one week. Aliquots were removed at 24 and 168 hours for sCD163 assay (n=6 per condition), with the level being normalised to the level in the original sample. Stored urine was then taken from the same patient and subjected aliquots to between 1 and 6 freeze-thaw cycles to assess the effect on sCD163 level.

### Statistical assessment

Analyses were performed using GraphPad Prism version 6.01 for Windows (GraphPad Software, San Diego California USA), SPSS (IBM, version 21, Amrok, USA) and R (R Foundation for Statistical Computing, version 3.1.1, Vienna, Austria). Data were expressed as median ± interquartile range and p-values <0.05 were considered significant. Differences between two groups were analysed using the Mann-Whitney U test, while differences between 3 or more groups were analysed using Kruskal-Wallis test with post-hoc Dunn's test. To assess the ability of sCD163 to predict active renal vasculitis over urine protein excretion rate, receiver operator characteristic (ROC) curves for sCD163, urine PCR and their combination were estimated using data from the combined cohorts. Note that the full cohort was not available for this analysis, so the optimum sCD163 cutoff was slightly different. Logistic regression was used to estimate the combination of sCD163 and urine protein excretion rate. Inference on differences between ROC curves was performed using bootstrap resampling. The point on the curves which maximised the sum of sensitivity and specificity was chosen as a cut-off for predicting active renal vasculitis. For PCR, the standard laboratory cut-off of 15mg/mmol was used. Optimum cut-offs were then used to predict active renal vasculitis in the validation cohort and estimate sensitivity, specificity, positive/negative predictive value, positive/negative likelihood ratios.

### Measuring levels of monocyte chemoattractant protein -1 (MCP -1)

Levels of MCP-1 were measured in a biological samples (urine, serum and biopsy) as described above for sCD163. MCP-1 can also be measured in a sample by ELISA and a set out in Lieberthal *et al.* (Lieberthal JG, Cuthbertson D, Carette S, Hoffman GS, Khalidi NA, Koening CL, Langford CA,Maksimowicz-McKinnon K, Seo P, Specks U, Ytterberg SR, Merkel PA, Monach PA; Vasculitis Clinical Research Consortium: urinary biomarkers in relapsing antineutrophil cytoplasmic antibody-associated vasculitis. J Rheumatol 40: 674-683, 2013).

### Results

### CD163 is expressed in the kidney of rats with EAV and appears in the urine during the evolution phase of renal vasculitis

Rats immunised with hMPO analysed at day 28 had proliferative GN and occasional early crescent formation (Figure 3). At day 56, rats had fibrinoid necrosis and crescentic glomerulonephritis, as described previously13, and CD163+ cells were present in both the glomerular and tubulointerstitial compartments (Figure 3B-H). To further phenotype the CD163+ cells, EAV kidney tissue was stained for CCR7 (macrophage subtype M1 marker) and CD206 (M2 marker, Figure 3I-K). CD163 did not co-localise with CCR7, but was variably co-localised with CD206, with CD163+CD206- cells also being present. Urinary sCD163 excretion was measured over a time course (Figure 3A) and surprisingly found that, although histological evidence of disease was most severe at day 56, urine sCD163 level was highest at day 28 (mean 3.6±1.1 ng/mmol). The level returned to that observed in control animals by day 56 (1.5±0.4ng/mmol) and continued to fall further by six months (0.5±0.1ng/mmol). The level of urine sCD163 was correlated with the degree of macrophage infiltration (Figure 3L).

### CD163 is highly expressed in the glomeruli of patients with vasculitis

To assess the level of CD163 expression in the kidney of patients with renal vasculitis we first measured CD163 mRNA in microdissected glomerular and tubulointerstitial compartments in diabetic nephropathy, minimal change disease, IgA nephropathy, focal and segmental glomerulosclerosis, membranous nephropathy, lupus nephritis and ANCA vasculitis (Figure 4). Of all conditions examined, CD163 was most highly expressed in the glomeruli and tubulointerstitium of patients with ANCA vasculitis (fold change 23.4x and 7.5x above control respectively, p<0.001), with increased expression also noted in diabetic nephropathy (fold change 5.2x above control, p<0.001), IgA nephropathy (3.4x above control, p<0.05) and lupus nephritis (7.4x above control, p<0.001). The degree of CD163 gene expression was also highest in the tubulointerstitial compartment of patients with ANCA vasculitis (7.5x above control, p<0.001), with a lesser elevation noted in lupus nephritis (2.0x above control, p<0.05). High expression of CD163 was observed in both glomeruli and interstitium of patients with active renal vasculitis. Interestingly, CD163 was observed in glomeruli that appeared unaffected based on histological markers. Patients with anti-GBM disease exhibited the highest CD163 expression in all 5 compartments scored, particularly in histologically unaffected glomerular tufts. CD163 expression was similar in MPO and PR3-ANCA positive patients. However, when patients were separated based on diagnosis rather than autoantibody specificity, patients with granulomatosis with polyangiitis (GPA) were found to have relatively low levels of kidney CD163 expression (Figure 4C). Very occasional CD163+ cells were noted in healthy kidney from live transplant donors (Figure 4E) and healed fibrous crescents exhibited very little CD163 staining (Figure 4I).

### The serum level of sCD163 in vasculitis patients is neither increased nor correlated with the urine sCD163 level

To assess whether the sCD163 level predicted vasculitic injury, the levels were measured in the serum of vasculitis patients (n=243, 59 active and 184 remission), healthy controls (n=57) and disease controls (n=37, Figure 8). There were no significant differences in the levels of serum sCD163 in active vasculitis patients (median 387ng/ml, interquartile range 283-613) compared to remission vasculitis patients (313ng/ml, 226-472), disease controls (298ng/ml, 236-475) and healthy controls (287ng/ml, 226-463) (Figure 8).

Furthermore, serum sCD163 levels did not correlate with urinary sCD163 levels (r=0.15) (Figure 8).

### Urinary sCD163 levels are elevated in active renal vasculitis

Urinary sCD163 was measured in an inception cohort of 177 subjects [14.6% with active renal vasculitis (n=26), 5.9% with active extra-renal vasculitis (n=11) and 79.4% in remission (n=140), Table 2]. In order to account for differences in the concentration of urine between individuals, all sCD163 values were normalised to urinary creatinine. Normalised urinary sCD163 levels were significantly elevated in patients with active renal vasculitis (0.56ng/mmol) compared to patients with active extra-renal vasculitis (0.12ng/mmol), remission renal vasculitis (0.11ng/mmol) and remission extra-renal vasculitis (O.lng/mmol) (Figure 5A). To assess whether this finding was a surrogate of haematuria (reflecting monocyturia), sCD163 was assayed in control urine spiked with serial dilutions of blood and correlated the result with urine dipstick analysis of haematuria. Addition of blood to urine only led to a rise in sCD163 level when the concentration of blood in urine was >2%. The urine appeared frankly blood-stained down to a concentration of 0.7% and was dipstick positive for blood down to 0.1%, levels at which sCD163 was undetectable (Figure 7C). In order to assess the stability of sCD163 in urine, samples were stored under various conditions and aliquots were taken for repeated analyses. There was no significant change in sCD163 level after one week of storage at room temperature, 4°C or -20°C, and the sCD163 level was stable for up to 4 freeze-thaw cycles. Freeze-thaw cycles beyond this caused a significant decline in sCD163 level (Figure 7A,B).

**Table 2 Demographic and clinical information for patients and controls. P-values show significance between patient groups in inception, internal validation and external validation cohorts. Age is presented as median and range; all other values are presented as n (%). GPA = granulomatosis with polyangiitis, MPA = microscopic polyangiitis, EGPA = eosinophilic granulomatosis with polyangiitis, GBM = glomerular basement membrane, eGFR = estimated glomerular filtration rate, ITU = Intensive therapy unit. *Comparing inception, Validation 1 and Validation 2 cohorts. -Comparing SVV cohorts.**

| | **Inceptio n** | **Validation cohort 1** | **Validation cohort 2** | **External Validation (n=52)** | | | **Dise ase Cont rols** | **Heal thy cont rols** | |
|---|---|---|---|---|---|---|---|---|---|
| | | | | ***Patie nts*** | ***Heal thy Cont* rols** | | | | p-**valu e** |
| **n** | 177 | 155 | 133 | 39 | 13 | | 865 | 55 | |
| **Age (median, range)** | 63.5 (21.2-90.2) | 60.3 (16.7-95.3) | 59.9 (18.1-91.0) | 58.6 (31.3 81.9) | 53.8 (34.6 - 64.1) | | 55.4 (16.6 - 87.3) | 52.1 | 0.03 |
| **Male, n** (%) | 87 (49) | 97 (62.6) | 80 (60.1) | 16 (41.1 ) | 6 (46.2 ) | | 411 (47.6 ) | 20 (36.3 ) | 0.00 04 |
| *Diagnosis, n* (%) | | | | | | | | | 0.07 |
| **GPA** | 101 (57.1) | 94 (60.6) | 57 (42.9) | 28 (71.8 ) | | **Glomerulonep hritis** | 30 | | |
| **MPA** | 54(31.1) | 49(31.6) | 59 (44.3) | 11 (28.2 ) | | Non **Glomerulonep hritis** | 54 | | |
| **EGPA** | 12 (6.8) | 6 (3.9) | 8 (6.0) | 0(0) | | **ITU:** sepsis+/- AKI | 32 | | |
| **Anti-GBM disease** | 3 (1.7) | 3 (1.9) | 5 (3.8) | 0 (0) | | **ITU: No sepsis** + **AKI** | 286 | | |
| **Double Positive** | 6 (3.4) | 3 (1.9) | 4 (3.0) | 0(0) | | **ITU: No sepsis** / **No AKI** | 463 | | |
| **Disease Characteristics, n** (%) | | | | | | | | | 0.01 * |
| **Active (renal)** | 26 (14.6) | 15 (9.7) | 33 (24.8) | 24 (61.5 ) | | | | | |
| **Active (extra-renal)** | 11 (5.9) | 9 (5.8) | 6 (4.5) | 3 (7.7) | | | | | |
| **Remission** | 140 (79.4) | 131 (84.5) | 94 (70.7) | 12 (30.8 ) | | | | | |
| **Kidney function, n (%)** | | | | | | | | | 0.3^{∼} |
| **eGFR <30** | 35 (19.8) | 34 (21.9) | 33 (24.8) | 14 (35.9 ) | | | 258 (29.8 ) | | |
| **eGFR 30-60** | 50 (28.2) | 49 (31.6) | 44 (33.1) | 10 (25.6 ) | | | 313 (36.2 ) | | |
| **eGFR >60** | 92 (52) | 72 (46.5) | 56 (42.1) | 15 (38.5 ) | | | 294 (34.0 ) | | |
| **Dialysis** | 9 (5.1) | 3 (1.9) | 12 (9.0) | N/A | | | 20 (2.3) | | |

### Validation of the tight association between elevated urine sCD163 and active renal vasculitis

Using the data from the inception cohort, a receiver-operator characteristic (ROC) curve was generated to identify active renal vasculitis in patients with a known diagnosis of vasculitis (Figure 5). The area under the ROC curve was 0.94 indicating excellent biomarker potential. This was used to define an optimum cut-off value of 0.3ng/ml/mmol and it was applied to the validation cohort 1, consisting of 155 vasculitis patients, 8 healthy controls and 18 disease controls, and validation cohort 2, consisting of 133 patients, 13 healthy controls and 39 disease controls. The validation cohort 1 provided virtually identical results to the inception cohort with sCD163 being significantly elevated in the urine of patients with active renal vasculitis compared to patients with active extra-renal vasculitis (p<0.0001), remission patients with previous renal (p<0.0001) or extra-renal (p<0.0001) vasculitis, disease controls (p<0.01) and healthy controls (p<0.0001, Figure 5B). The validation cohort 2 provided virtually identical results to the inception cohort, with sCD163 significantly elevated in the urine of patients with active renal vasculitis compared to patients with active extra-renal vasculitis and remission patients with previous renal or extra-renal vasculitis (Figure 5C).

Using the cut-off value of 0.3 ng/ml/mmol, 87% and 73% of active patients with renal vasculitis were positive, compared to 0% (active extra-renal vasculitis), 1.2% (remission renal vasculitis), 4% (remission extra-renal vasculitis), 9% (disease controls) and 0% (healthy controls). Biomarker statistics for the combined cohorts are summarised in Table 3. Conversely, none of the patients with active extra-renal vasculitis were positive. False positive rates in remission patients were 1.5% and 3.4% respectively for patients with previous renal and extra-renal vasculitis. Stratification of values by ANCA specificity (PR3 v MPO) revealed no difference in the ability of sCD163 to identify active renal vasculitis (Figure 8). Biomarker statistics for diagnosis of active renal vasculitis within the overall vasculitis cohorts are summarised in Table 4. Notable values are specificity of 96% and positive likelihood ratio of 20.8. The non-normalised (to creatinine) urine sCD163 values also performed well, although specificity (92 %) and positive likelihood ratio (10.0) were less than for the normalised values (Table 4). In individuals where sCD163 was measured in serial samples following presentation with active disease the level fell rapidly with treatment, and remained low. In those with active extra-renal vasculitis, the level was low at all time points (Figure 5E). To assess the relationship between renal CD163 protein expression and the urine sCD163 level, 25 cases were identified with renal tissue obtained at approximately the same time as the test urine sample. Of these, sufficient glomerular tissue was available for analysis from 17 patients and extra-glomerular tissue from 20. There was a correlation between the number of CD163 positive cells in glomeruli and the linked level of urine sCD163 (Figure 9). The correlation with extra-glomerular staining was weaker.

**Table 3. Generation of optimum cut-off in inception cohort, with application to validation cohort. Statistics were determined by first analysing data from the inception cohort to define optimum cut-off, and applying this to the validation cohort. AUC = area under ROC curve, PPV = positive predictive value, NPV = negative predictive value.**

| | AUC | Cutoff | Sensitivity | Specificity | PPV | NPV | PLR | NLR |
|---|---|---|---|---|---|---|---|---|
| *Inception* | | | | | | | | |
| sCD163 normalised | 0.96 | 0.3 | 0.96 | 0.94 | 0.69 | 0.99 | 14.7 | 0.05 |

| *Validation* | | | | | | | | |
|---|---|---|---|---|---|---|---|---|
| sCD163 normalised | 0.97 | 0.3 | 0.87 | 0.98 | 0.81 | 0.99 | 43.5 | 0.13 |

| *Validation 2* | | | | | | | | |
|---|---|---|---|---|---|---|---|---|
| sCD163 normalised | 0.94 | 0.3 | 0.73 | 0.98 | 0.92 | 0.92 | 36.5 | 0.28 |

**Table 4. Biomarker attributes of normalised and non-normalised urine sCD163 for discriminating active renal vasculitis from remission. Statistics were determined by analysing combined data from both the inception and validation cohorts. AUC = area under ROC curve, PPV = positive predictive value, NPV = negative predictive value.**

| | AUC | Cut-off | Sensitivity | Specificity | PPV | NPV | PLR | NLR |
|---|---|---|---|---|---|---|---|---|
| SCD163 normalised | 0.93 | 0.3 | 0.83 | 0.96 | 0.80 | 0.97 | 20.75 | 0.17 |
| SCD163 non-normalised | 0.91 | 1.3 | 0.80 | 0.92 | 0.64 | 0.96 | 10.0 | 0.22 |

### External validation of the association between elevated urine sCD163 and active renal vasculitis

An independent Dutch cohort comprising 52 subjects (39 SVV patients and 13 healthy controls) was interrogated to further validate the findings. These analyses confirmed the ability of urinary sCD163 to identify active renal disease in SVV patients. sCD163 was significantly elevated in the urine of patients with active renal vasculitis compared to remission patients with previous renal involvement and healthy controls (Figure 6B). Using the cut-off value of 0.3ng/mmol, 79% of patients with active renal vasculitis were positively diagnosed. No false positive cases were seen in patients with active extra-renal vasculitis, remission renal vasculitis or healthy controls.

### Patients with kidney disease without active crescentic glomerulonephritis do not present with elevated urinary sCD163

In order to examine the specificity of elevated urinary sCD163 for active renal vasculitis urine sCD163 level was measured in a series of disease control groups (Figure 6A, Table 2). Elevated urine sCD163 levels (above cut-off of 0.3ng/mmol creatinine) were identified in a small number of critically ill patients in the intensive care unit, with 6.3% of patients with sepsis and acute kidney injury (AKI), 14.7% of patients with AKI but no sepsis and 7.8% of patients with no AKI or sepsis testing positive. Urinary sCD163 was not significantly elevated in a cohort of disease controls without glomerulonephritis (n=54), or in healthy controls (n=55). However, one quarter of disease controls with non-vasculitic glomerulonephritis, in whom variable degrees of macrophage infiltration would be expected, had an elevated level (n=30).

### Urine sCD163 is a significantly better biomarker for active renal vasculitis than urine protein excretion rate

Using the internal validation cohorts, the utility of urine sCD163 was tested in the identification of active renal vasculitis against urine protein:creatinine ratio (PCR), using a PCR cut-off of 15mg/mmol (Table 5). On its own, urine sCD163 was superior by all measures of biomarker performance including sensitivity and specificity which acts a measure of false positive rate. Combining urine sCD163 with urine PCR did not provide a statistically significant improvement in biomarker performance versus the use of sCD163 alone. In a small subset of individuals for whom samples were available from an incident histologically-proven renal flare the biomarker precision of urine sCD163, c-reactive protein, haematuria and ANCA titre were compared (Table 6). Urine sCD163 was superior to the other three biomarkers by all measures of biomarker performance.

**Table 5. Biomarker comparison of urine sCD163 with urine PCR and combination sCD163/PCR for discriminating active renal vasculitis from remission. Statistics were determined by analysing data from a subset of cases from the combined cohort. The bootstrap test compared the AUC sCD163 and PCR (*). AUC = area under the ROC curve, PCR = protein/creatinine ratio, PPV = positive predictive value, NPV = negative predictive value, PLR = positive likelihood ratio, NLR = negative likelihood ratio.**

| | **AUC** | **Cutoff** | **Sensitivity** | **Specificity** | **PPV** | **NPV** | **PLR** | **NLR** |
|---|---|---|---|---|---|---|---|---|
| **sCD163 normalised** | 0.91 | 0.3 | 0.83 | 0.97 | 0.89 | 0.95 | 30.6 | 0.17 |
| **PCR** | 0.68 | 15 | 0.97 | 0.39 | 0.3 | 0.97 | 1.6 | 0.08 |
| **sCD163 + PCR** | 0.93 | | 0.8 | 0.97 | 0.89 | 0.95 | 29.33 | 0.21 |
| ***Bootstrap P-value*** | 0.07 | | | | | | | |

**Table 6. Biomarker comparison of urine sCD163 with CRP, urinalysis and ANCA for discriminating biopsy-proven renal flare from remission. The 4 biomarkers in question were compared for their ability to identify those with a biopsy-proven renal flare (n=7), all of whom had elevated urine sCD163. In the subset of patients suffering a biopsy-proven flare (n=7) *or turning positive from negative. PPV/NPV = Positive and negative predictive value**

| | **Accuracy (95% Interval)** | **Sensitivity** | **Specificity** | **PPV** | **NPV** |
|---|---|---|---|---|---|
| Normalised sCD163 > 0.3 | 0.97 (0.83 - 1) | 1 | 0.96 | 0.88 | 1 |
| CRP > 5 | 0.65 (0.45 - 0.81) | 1 | 0.54 | 0.39 | 1 |
| New/increased haematuria | 0.87 (0.7 - 0.96) | 0.43 | 1 | 1 | 0.86 |
| >4-fold rise in ANCA titre* | 0.84 (0.66 - 0.95) | 0.57 | 0.92 | 0.67 | 0.88 |

### Urine sCD163 levels fall with induction of remission

To assess the time course of urinary sCD163 excretion, the level was measured sequentially in a subset of recruits at several time points following presentation with active disease (Figure 5E). In patients with active renal vasculitis, the level fell rapidly with treatment, and remained low. In those with active extra-renal vasculitis, the level remained low at all time points.

### Assessment of the utility of urine sCD163 in tracking glomerular inflammation during induction treatment for CGN

Inclusion criteria: Patients treated for *de novo* or relapsing CGN with corticosteroids plus either daily oral cyclophosphamide, pulsed intravenous cyclophosphamide or rituximab. These patients may have either ANCA vasculitis, anti-GBM disease or lupus nephritis. The majority will have ANCA vasculitis, but that the biological signal will be similar irrespective of the cause of CGN. The glomerulonephritis should be biopsy proven; in the absence of histological proof, a clinical picture of RPGN (rapidly rising serum creatinine associated with blood and protein on urine dipstick with appropriate positive serological tests) will be acceptable. The aim is to improve upon current measures of identifying when to switch from induction to maintenance therapy (haematuria and proteinuria, see Figure 2), which continue to improve long after the point at which this switch is conventionally made.

Exclusion criteria: Anuric. Patients on dialysis that pass urine will be included.

Readouts: Median and interquartile range of urine sCD163 at diagnosis, prior to each dose of cyclophosphamide or at monthly intervals after treatment with rituximab / during treatment with oral cyclophosphamide, at the time of switch to maintenance treatment and one year after enrolment (9-15 samples per recruit). The actuarial time to nadir urine sCD163 will be estimated using survival analysis.

Comparators: Samples will be taken at the same time for estimation of urine protein:creatinine ratio (PCR), urine dipstick for blood and serum creatinine; these will be compared to urine sCD163 at each time point. The area under the sCD163 curve will be correlated with the initial crescent fraction, where available, and with the starting eGFR, as determined by the CKD-Epi equation [Michels, W.M., et al., Performance of the Cockcroft-Gault, MDRD, and new CKD-EPI formulas in relation to GFR, age, and body size. Clin J Am Soc Nephrol, 2010. 5(6): p. 1003-9.]. The actuarial time to nadir sCD163 will be compared to time to nadir proteinuria and serum creatinine level, and time to disappearance of dipstick haematuria using Cox regression analysis.

The delta eGFR will be compared between those with a positive urine sCD163 assay at the time of completion of induction therapy versus those with a negative assay, with the same analysis done for the additional comparator biomarkers. A similar analysis using sCD163 as a continuous variable will correlate it with delta eGFR.

It is expected that the experiments will define a 95% confidence interval for the urine sCD163 value over the course of induction treatment for CGN and will allow to determine the time course of this biomarker compared to urine protein, urine blood and serum creatinine.

### Results from measurements of MCP-1

It is expected to find elevated levels of MCP-1 in the urine, but not the serum of patients with active or persistent renal vasculitis. It is also expected to find levels of MCP-1 in the urine to decline with treatment for the same. Elevated levels of MCP-1 discriminate active renal vasculitis with a high specificity and sensitivity.

A urinary biomarker that could reliably identify active renal vasculitis in patients already diagnosed with SVV would reduce the need for expensive and invasive kidney biopsies. In this context, several protein biomarkers have been investigated as biomarkers of active disease in vasculitis, including alpha-1 acid glycoprotein, kidney injury molecule-1, fractalkine, neutrophil gelatinase-associated lipocalin and monocyte chemoattractant protein-1 (MCP-1). sCD163 is a highly stable protein, making it an attractive clinical biomarker. In whole blood it is stable for 24 hours and 48 hours at room temperature and 4°C respectively, while in plasma it is stable for weeks at 4°C and several years at -20°C. Similarly, in urine it was found that sCD163 is stable for at least one week at room temperature. This stability means that variations in the collection and processing of samples are unlikely to be a determining factor in the assay result. This represents a significant advantage over MCP1, which degrades quickly after frozen storage and freeze thaw cycles. MCP1 is actively produced by intrinsic renal cells and leukocytes in response to inflammatory stimuli, resulting in monocyte chemoattraction, cells which themselves are likely to express CD163. Therefore, MCP-1 and sCD163 measurements reflect subtly different elements of a similar process; their use in combination may further increase diagnostic precision.

The excellent biomarker characteristics of urinary sCD163 in identifying active renal vasculitis, the availability of high quality detection antibody and its stability during sample storage suggest that this biomarker could now be developed for clinical use. Most biomarker development programmes rely upon panels of proteins or metabolites; the ability of urine sCD163 alone (or normalised to creatinine) to identify active renal vasculitis suggests that it is ideal for development as a point-of-care dipstick test and may have additional use in tracking induction therapy response, allowing more tailored and less toxic treatment regimens, or potentially to triage patients with undifferentiated acute kidney injury in settings where there may be delay in obtaining serological or histological diagnosis.

In the specification the terms "comprise, comprises, comprised and comprising" or any variation thereof and the terms "include, includes, included and including" or any variation thereof are considered to be totally interchangeable and they should all be afforded the widest possible interpretation and vice versa.

The invention is not limited to the embodiments hereinbefore described but may be varied in both construction and detail.

## Claims

1. A method for identifying an individual with active crescentic glomerulonephritis (CGN), the method comprising the step of assaying a biological sample obtained from the individual for the presence of an elevated level of soluble CD 163 (sCD 163) when compared to levels in a reference sample, wherein an elevated level of sCD163 when compared to levels in a reference sample correlates with the individual having active CGN,
wherein the biological sample is urine, and
the CGN is caused by small vessel vasculitis (SW) or anti-glomerular basement membrane (anti-GBM) antibody disease.

2. A method according to Claim 1, further comprising the step of assaying the biological sample obtained from the individual for the presence of an elevated level of monocyte chemoattractant protein- 1 (MCP-1) when compared to levels in a reference sample, wherein an elevated level of MCP-1 when compared to levels in a reference sample, in combination with an elevated level of sCD163 when compared to levels in a reference sample, correlates with the individual having active CGN.

3. A method for assessing whether an individual should continue to receive treatment for CGN, the method comprising the step of assaying a biological sample obtained from the individual for the presence of an elevated level of soluble CD 163 (sCD163) when compared to levels in a reference sample, wherein an elevated level of sCD163 when compared to levels in a reference sample correlates with the individual being suitable for continuance of the treatment, wherein the biological sample is urine, and
the CGN is caused by small vessel vasculitis (SW) or anti-glomerular basement membrane (anti-GBM) antibody disease.

4. A method according to Claim 3, further comprising the step of assaying the biological sample obtained from the individual for the presence of an elevated level of monocyte chemoattractant protein- 1 (MCP-1) when compared to levels in a reference sample, wherein an elevated level of MCP-1 when compared to levels in a reference sample, in combination with an elevated level of sCD163 when compared to levels in a reference sample, correlates with the individual being suitable for continuance of the treatment.

5. A method according to Claim 3 or Claim 4 in which the treatment consists of glucocorticoid treatment and/or immunosuppressant treatment.

6. A method for distinguishing CGN flare from other causes of clinical decline in an individual known previously to have CGN, the method comprising the step of assaying a biological sample obtained from the individual for the presence of an elevated level of soluble CD 163 (sCD163) when compared to levels in a reference sample, wherein an elevated level of sCD163 when compared to levels in a reference sample correlates with the individual suffering from CGN flare and not from other causes of clinical decline,
wherein the biological sample is urine, and
the CGN flare is caused by small vessel vasculitis (with and without the presence of anti-neutrophil cytoplasm antibodies (ANCA)) or anti-glomerular basement membrane (anti-GBM) antibody disease.

7. A method according to Claim 6, further comprising the step of assaying the biological sample obtained from the individual for the presence of an elevated level of monocyte chemoattractant protein- 1 (MCP-1) when compared to levels in a reference sample, wherein an elevated level of MCP-1 when compared to levels in a reference sample, in combination with elevated levels of sCD163 when compared to levels in a reference sample, correlates with the individual suffering from CGN flare and not from other causes of clinical decline.

8. A method according to Claim 6 or Claim 7, in which the other causes of clinical decline comprise ischemic renal injury, interstitial nephritis, secondary to paraprotein deposition, drug toxicity, myoglobinuric tubular injury, malignancy, acute tubular necrosis, urine infection, haemolytic uremic syndrome/thrombotic thrombocytopenic purpura or fibrillary glomerulonephritis.

9. A method according to any one of the preceding claims in which the reference sample is an individual identified as having kidney disease not caused by SW, acute active CGN or other forms of CGN.

10. Glucocorticoid or immunosuppressive therapy for use in a method for treating an individual with established crescentic glomerulonephritis (CGN) or recurrence of CGN, the method comprising the steps of assaying a biological sample obtained from the individual for the presence of an elevated level of soluble CD 163 (sCD163) when compared to levels in a reference sample, identifying the individual for treatment if the biological sample shows an elevated level of sCD163 when compared to levels in a reference sample, and treating the individual with a glucocorticoid and/or an immunosuppressive therapy,
wherein the biological sample is urine,
the CGN is caused by small vessel vasculitis (SW) or anti-glomerular basement membrane (anti-GBM) antibody disease,
the glucocorticoid is selected from prednisolone, methyl-prednisolone, hydrocortisone or dexamethasone, and
immunosuppressive therapy is selected from cyclophosphamide, rituximab, mycophenolate mofetil, azathioprine or methotrexate.

11. A method according to any preceding Claim in which the assaying step employs an in vitro diagnostic apparatus configured to detect an elevated level of a marker selected from sCD163, and optionally MCP-1, in a biological sample, and in which the method comprises a step of assaying the biological sample with the in vitro diagnostic apparatus.

12. A method according to Claim 11 in which the in vitro diagnostic apparatus comprises an antibody or antibody fragment capable of specifically binding to a protein marker selected from sCD163 and optionally MCP-1 immobilised to a support, in which the method comprises the steps of contacting the biological sample with the antibody or antibody fragment under conditions that allow an immuno specific protein-antibody or protein-antibody fragment binding reaction to occur, and detection of an immuno specific protein-antibody or protein-antibody fragment binding reaction.

13. A method according to Claim 11 or 12 in which the in vitro diagnostic apparatus is selected from an ELISA kit and a dipstick kit.

14. A method according to Claims 11 to 13, in which the in vitro diagnostic apparatus is a dipstick kit comprising: a dipstick having the antibody or antibody fragment capable of specifically binding to sCD163 and optionally MCP-1 immobilised thereon; one or more reagents capable of detecting the immuno specific protein-antibody or protein- antibody fragment binding reaction and emitting a detectable signal; and a correlating device for correlating the detectable signal with an elevated level of sCD163 and optionally MCP-1.

15. A method according to Claim 14, in which the detectable signal is a colour change and in which the correlating device is a colour chart.

## Patentansprüche

1. Verfahren zum Identifizieren einer Person mit aktiver Glomerulonephritis mit Halbmondbildung (CGN), wobei das Verfahren den Schritt des Untersuchens einer biologischen Probe, die von der Person erhalten wurde, auf das Vorhandensein eines erhöhten Gehalts an löslichem CD163 (sCD163) im Vergleich zu Gehalten in einer Referenzprobe umfasst, wobei ein erhöhter Gehalt an sCD163 im Vergleich zu Gehalten in einer Referenzprobe damit korreliert, dass die Person aktive CGN hat,
wobei die biologische Probe Urin ist, und
die CGN durch eine Vaskulitis der kleinen Gefäße (SVV) oder eine antiglomeruläre Basalmembran (anti-GBM) Antikörper-Erkrankung verursacht wird.

2. Verfahren nach Anspruch 1, ferner umfassend den Schritt des Untersuchens der biologischen Probe, die von der Person erhalten wurde, auf das Vorhandensein eines erhöhten Gehalts an monocyte chemoattractant protein-1 (MCP-1) im Vergleich zu Gehalten in einer Referenzprobe, wobei ein erhöhter Gehalt von MCP-1 im Vergleich zu Gehalten in einer Referenzprobe in Kombination mit einem erhöhten Gehalt von sCD163 im Vergleich zu Gehalten in einer Referenzprobe damit korreliert, dass die Person aktive CGN hat.

3. Verfahren zum Beurteilen, ob eine Person weiterhin eine CGN-Behandlung erhalten soll, wobei das Verfahren den Schritt des Untersuchens einer biologischen Probe, die von der Person erhalten wurde, auf das Vorhandensein eines erhöhten Gehalts an löslichem CD163 (sCD163) im Vergleich zu Gehalten in einer Referenzprobe umfasst, wobei ein erhöhter Gehalt an sCD163 im Vergleich zu Gehalten in einer Referenzprobe damit korreliert, dass die Person zur Fortsetzung der Behandlung geeignet ist,
wobei die biologische Probe Urin ist, und
die CGN durch eine Vaskulitis der kleinen Gefäße (SVV) oder eine antiglomeruläre Basalmembran (anti-GBM) Antikörper-Erkrankung verursacht wird.

4. Verfahren nach Anspruch 3, ferner umfassend den Schritt des Untersuchens der biologischen Probe, die von der Person erhalten wurde, auf das Vorhandensein eines erhöhten Gehalts an monocyte chemoattractant protein-1 (MCP-1) im Vergleich zu Gehalten in einer Referenzprobe, wobei ein erhöhter Gehalt von MCP-1 im Vergleich zu Gehalten in einer Referenzprobe in Kombination mit einem erhöhten Gehalt von sCD163 im Vergleich zu Gehalten in einer Referenzprobe damit korreliert, dass die Person zur Fortsetzung der Behandlung geeignet ist.

5. Verfahren nach Anspruch 3 oder Anspruch 4, bei dem die Behandlung aus einer Glukokortikoidbehandlung und/oder einer immunsuppressiven Behandlung besteht.

6. Verfahren zum Unterscheiden eines Aufflammens von CGN von anderen Ursachen für eine klinische Verschlechterung bei einer Person, von der vorher bekannt ist, dass sie CGN hat, wobei das Verfahren den Schritt des Untersuchens einer biologischen Probe, die von der Person erhalten wurde, auf das Vorhandensein eines erhöhten Gehalts an löslichem CD163 (sCD163) im Vergleich zu Gehalten in einer Referenzprobe umfasst, wobei ein erhöhter Gehalt an sCD163 im Vergleich zu Gehalten in einer Referenzprobe damit korreliert, dass die Person an einem Aufflammen von CGN leidet und nicht an anderen Ursachen für eine klinische Verschlechterung,
wobei die biologische Probe Urin ist, und
das Aufflammen von CGN durch eine Vaskulitis der kleinen Gefäße (mit und ohne Vorhandensein von antineutrophilen Zytoplasma-Antikörpern (ANCA)) oder eine antiglomeruläre Basalmembran (anti-GBM) Antikörper-Erkrankung verursacht wird.

7. Verfahren nach Anspruch 6, ferner umfassend den Schritt des Untersuchens der biologischen Probe, die von der Person erhalten wurde, auf das Vorhandensein eines erhöhten Gehalts an monocyte chemoattractant protein-1 (MCP-1) im Vergleich zu Gehalten in einer Referenzprobe, wobei ein erhöhter Gehalt an MCP-1 im Vergleich zu Gehalten in einer Referenzprobe, in Kombination mit erhöhten Gehalten an sCD163 im Vergleich zu Gehalten in einer Referenzprobe, damit korreliert, dass die Person an einem Aufflammen von CGN leidet und nicht an anderen Ursachen für eine klinische Verschlechterung.

8. Verfahren nach Anspruch 6 oder Anspruch 7, bei dem die anderen Ursachen für klinische Verschlechterung ischämische Nierenverletzung, interstitielle Nephritis, sekundär zur Paraproteinablagerung, Medikamententoxizität, myoglobinurische tubuläre Verletzung, Malignität, akute tubuläre Nekrose, Urininfektion, Hämolytisch-urämisches Syndrom / Thrombotisch-thrombocytopenische Purpura oder fibrilläre Glomerulonephritis umfassen.

9. Verfahren nach einem der vorhergehenden Ansprüche, bei dem die Referenzprobe eine Person ist, die eine Nierenerkrankung hat, die nicht durch SVV, akute aktive CGN oder andere Formen von CGN verursacht wird.

10. Glukokortikoid- oder immunsuppressive Therapie zur Verwendung in einem Verfahren zur Behandlung einer Person mit etablierter Glomerulonephritis mit Halbmondbildung (CGN) oder wiederauftretender CGN, wobei das Verfahren die Schritte des Untersuchens einer biologischen Probe, die von der Person erhalten wurde, auf das Vorhandensein eines erhöhten Gehalts an löslichem CD163 (sCD163) im Vergleich zu Gehalten in einer Referenzprobe, Identifizieren der zu behandelnden Person, wenn die biologische Probe im Vergleich zu Gehalten in einer Referenzprobe einen erhöhten sCD163-Gehalt aufweist, und Behandeln der Person mit einem Glukokortikoid und/oder einer immunsuppressiven Therapie umfasst,
wobei die biologische Probe Urin ist,
die CGN durch eine Vaskulitis der kleinen Gefäße (SVV) oder eine antiglomeruläre Basalmembran (anti-GBM) Antikörper-Erkrankung verursacht wird,
das Glukokortikoid aus Prednisolon, Methyl-Prednisolon, Hydrocortison oder Dexamethason ausgewählt ist, und
die immunsuppressive Therapie aus Cyclophosphamid, Rituximab, Mycophenolatmofetil, Azathioprin oder Methotrexat ausgewählt ist.

11. Verfahren nach einem vorhergehenden Anspruch, bei dem der Untersuchungsschritt eine in-vitro-Diagnosevorrichtung verwendet, die konfiguriert ist, um einen erhöhten Gehalt eines Markers, ausgewählt aus sCD163, und optional MCP-1, in einer biologischen Probe nachzuweisen, und bei dem das Verfahren einen Schritt des Untersuchens der biologischen Probe mit der in-vitro-Diagnosevorrichtung umfasst.

12. Verfahren nach Anspruch 11, bei dem die in-vitro-Diagnosevorrichtung einen Antikörper oder ein Antikörperfragment umfasst, die in der Lage sind, spezifisch an einen Proteinmarker zu binden, der aus sCD163, und optional MCP-1, ausgewählt ist, und die auf einem Träger immobilisiert sind, wobei das Verfahren die Schritte des Kontaktierens der biologischen Probe mit dem Antikörper oder Antikörperfragment unter Bedingungen, die es ermöglichen, dass eine immunspezifische Protein-Antikörper- oder Protein-Antikörperfragment-Bindungsreaktion stattfindet, und des Nachweises einer immunspezifischen Protein-Antikörper- oder Protein-Antikörperfragment-Bindungsreaktion umfasst.

13. Verfahren nach Anspruch 11 oder 12, bei dem die in-vitro-Diagnosevorrichtung aus einem ELISA-Kit und einem Teststreifen-Kit ausgewählt ist.

14. Verfahren nach den Ansprüchen 11 bis 13, bei dem die in-vitro-Diagnosevorrichtung ein Teststreifen-Kit ist, umfassend: einen Teststreifen mit dem Antikörper oder Antikörperfragment darauf immobilisiert, die in der Lage sind, spezifisch an sCD163, und optional MCP-1, zu binden; ein oder mehrere Reagenzien, die in der Lage sind, die immunspezifische Protein-Antikörper- oder Protein-Antikörper-Fragment-Bindungsreaktion nachzuweisen und ein nachweisbares Signal abzugeben; und eine Korrelationsvorrichtung zum Korrelieren des nachweisbaren Signals mit einem erhöhten Gehalt von sCD163, und optional MCP-1.

15. Verfahren nach Anspruch 14, bei dem das nachweisbare Signal eine Farbänderung ist und bei dem die Korrelationsvorrichtung eine Farbskala ist.

## Revendications

1. Méthode destinée à identifier un individu souffrant d'une glomérulonéphrite à croissants (CGN) active, la méthode comprenant l'étape consistant à doser un échantillon biologique obtenu auprès de l'individu pour la présence d'un taux élevé de CD 163 soluble (sCD163) par rapport à des taux dans un échantillon de référence, où un taux élevé de sCD163 par rapport à des taux dans un échantillon de référence est corrélé avec le fait que l'individu souffre d'une CGN active,
où l'échantillon biologique est constitué d'urine, et
la CGN est provoquée par une vascularite des petits vaisseaux (SVV) ou une maladie des anticorps anti-membrane basale glomérulaire (anti-GBM).

2. Méthode selon la revendication 1, comprenant en outre l'étape consistant à doser l'échantillon biologique obtenu auprès de l'individu pour la présence d'un taux élevé de protéine 1 chimio-attractive des monocytes (MCP-1) par rapport à des taux dans un échantillon de référence, où un taux élevé de MCP-1 par rapport à des taux dans un échantillon de référence, en combinaison avec un taux élevé de sCD163 par rapport à des taux dans un échantillon de référence, est corrélé avec le fait que l'individu souffre d'une CGN active.

3. Méthode destinée à évaluer si un individu doit poursuivre un traitement contre la CGN, la méthode comprenant l'étape consistant à doser un échantillon biologique obtenu auprès de l'individu pour la présence d'un taux élevé de CD 163 soluble (sCD163) par rapport à des taux dans un échantillon de référence, où un taux élevé de sCD163 par rapport à des taux dans un échantillon de référence est corrélé avec le fait que l'individu est convenable pour la poursuite du traitement,
où l'échantillon biologique est constitué d'urine, et
la CGN est provoquée par une vascularite des petits vaisseaux (SVV) ou une maladie des anticorps anti-membrane basale glomérulaire (anti-GBM).

4. Méthode selon la revendication 3, comprenant en outre l'étape consistant à doser l'échantillon biologique obtenu auprès de l'individu pour la présence d'un taux élevé de protéine 1 chimio-attractive des monocytes (MCP-1) par rapport à des taux dans un échantillon de référence, où un taux élevé de MCP-1 par rapport à des taux dans un échantillon de référence, en combinaison avec un taux élevé de sCD163 par rapport à des taux dans un échantillon de référence, est corrélé avec le fait que l'individu est convenable pour la poursuite du traitement.

5. Méthode selon la revendication 3 ou la revendication 4, dans laquelle le traitement est constitué d'un traitement à base de glucocorticoïdes et/ou d'un traitement à base d'immunosuppresseurs.

6. Méthode destinée à distinguer une poussée de CGN d'autres causes de déclin clinique chez un individu connu précédemment comme souffrant d'une CGN, la méthode comprenant l'étape consistant à doser un échantillon biologique obtenu auprès de l'individu pour la présence d'un taux élevé de CD 163 soluble (sCD163) par rapport à des taux dans un échantillon de référence, où un taux élevé de sCD163 par rapport à des taux dans un échantillon de référence est corrélé avec le fait que l'individu souffre d'une poussée de CGN et non pas d'autres causes de déclin clinique,
où l'échantillon biologique est constitué d'urine, et
la poussée de CGN est provoquée par une vascularite des petits vaisseaux (avec ou sans la présence d'anticorps anti-cytoplasme des polynucléaires neutrophiles (ANCA)) ou une maladie des anticorps anti-membrane basale glomérulaire (anti-GBM).

7. Méthode selon la revendication 6, comprenant en outre l'étape consistant à doser l'échantillon biologique obtenu auprès de l'individu pour la présence d'un taux élevé de protéine 1 chimio-attractive des monocytes (MCP-1) par rapport à des taux dans un échantillon de référence, où un taux élevé de MCP-1 par rapport à des taux dans un échantillon de référence, en combinaison avec un taux élevé de sCD163 par rapport à des taux dans un échantillon de référence, est corrélé avec le fait que l'individu souffre d'une poussée de CGN et non pas d'autres causes de déclin clinique.

8. Méthode selon la revendication 6 ou la revendication 7, dans laquelle les autres causes de déclin clinique comprennent une lésion rénale ischémique, la néphrite interstitielle, secondaire à un dépôt de para-protéines, une toxicité médicamenteuse, une lésion tubulaire myoglobinurique, une malignité, une nécrose tubulaire aiguë, une infection urinaire, un purpura thrombotique thrombocytopénique/syndrome hémolytique et urémique, ou une glomérulonéphrite fibrillaire.

9. Méthode selon l'une quelconque des revendications précédentes, dans laquelle l'échantillon de référence est un individu identifié comme souffrant d'une maladie rénale non provoquée par une SVV, une CGN active aiguë ou d'autres formes de CGN.

10. Thérapie à base de glucocorticoïdes ou d'immuno-suppresseurs pour une utilisation dans une méthode destinée au traitement d'un individu souffrant d'une glomérulonéphrite à croissants (CGN) établie ou d'une récurrence de CGN, la méthode comprenant les étapes consistant à doser un échantillon biologique obtenu auprès de l'individu pour la présence d'un taux élevé de CD 163 soluble (sCD163) par rapport à des taux dans un échantillon de référence, identifier l'individu pour un traitement si l'échantillon biologique présente un taux élevé de sCD163 par rapport à des taux dans un échantillon de référence, et traiter l'individu par une thérapie à base de glucocorticoïdes ou d'immuno-suppresseurs,
où l'échantillon biologique est constitué d'urine,
la CGN est provoquée par une vascularite des petits vaisseaux (SVV) ou une maladie des anticorps anti-membrane basale glomérulaire (anti-GBM),
le glucocorticoïde est choisi parmi la prednisolone, la méthylprednisolone, l'hydroxycortisone ou la dexaméthasone, et
la thérapie à base d'immunosuppresseurs est choisie parmi le cyclophosphamide, le rituximab, le mycophénolate mofétil, l'azathioprine ou le méthotrexate.

11. Méthode selon l'une quelconque des revendications précédentes, dans laquelle l'étape de dosage emploie un appareil de diagnostic in vitro configuré afin de détecter un taux élevé d'un marqueur choisi parmi sCD163, et éventuellement MCP-1, dans un échantillon biologique, et la méthode comprenant une étape consistant à doser l'échantillon biologique dans l'appareil de diagnostic in vitro.

12. Méthode selon la revendication 11, dans laquelle l'appareil de diagnostic in vitro comprend un anticorps ou un fragment d'anticorps capable de se fixer spécifiquement à un marqueur protéique choisi parmi sCD163 et éventuellement MCP-1 immobilisé sur un support, la méthode comprenant les étapes consistant à mettre en contact l'échantillon biologique avec l'anticorps ou le fragment d'anticorps dans des conditions permettant l'obtention d'une réaction de fixation immunospécifique protéine-anticorps ou protéine-fragment d'anticorps, et détecter une réaction de fixation immunospécifique protéine-anticorps ou protéine-fragment d'anticorps.

13. Méthode selon la revendication 11 ou 12, dans laquelle l'appareil de diagnostic in vitro est choisi parmi un kit ELISA et un kit de bandelette réactive.

14. Méthode selon les revendications 11 à 13, dans laquelle l'appareil de diagnostic in vitro est un kit de bandelette réactive comprenant : une bandelette réactive ayant, immobilisé sur celle-ci, l'anticorps ou le fragment d'anticorps capable de se fixer spécifiquement à sCD163 et éventuellement MCP-1 ; un ou plusieurs réactifs capables de détecter la réaction de fixation immunospécifique protéine-anticorps ou protéine-fragment d'anticorps et émettant un signal détectable ; et un dispositif de corrélation destiné à la corrélation du signal détectable avec un taux élevé de sCD163 et éventuellement de MCP-1.

15. Méthode selon la revendication 14, dans laquelle le signal détectable est un changement de couleur et dans laquelle le dispositif de corrélation est un nuancier.
